# EUROPÄISCHE PATENTSCHRIFT

(11) **EP 0 512 432 B1**
(45) Veröffentlichungstag und Bekanntmachung des Hinweises auf die Patenterteilung: **29.07.1998**
(21) Anmeldenummer: 92107437.3
(22) Anmeldetag: 30.04.1992
(51) Int. Cl.: C07D 209/48, C11D 3/00, C07D 207/408

(54) **Imidocarbonsäureaktivatoren und Sulfimidocarbonsäureaktivatoren, Verfahren zu deren Herstellung und deren Verwendung**
Imido- and sulfimido carbonic acid activators, process for their preparation and their use
Activateurs imido- et sulfimidocarboxylique, procédé pour leur préparation et leur utilisation

(30) Priorität: 04.05.1991 DE 4114583
(43) Veröffentlichungstag der Anmeldung: 11.11.1992
(73) Patentinhaber: Clariant GmbH, 65929 Frankfurt am Main (DE)
(72) Erfinder: Jaekel, Frank, Dr., W-632 Bad Soden am Taunus (DE); Reinhardt, Gerd, Dr., W-6233 Kelkheim (Taunus) (DE); Müller, Wolf-Dieter, Dr., W-6238 Hofhheim am Taunus (DE)

(56) Entgegenhaltungen:
- EP-A- 0 349 940
- US-A- 4 980 482
- CHEMICAL ABSTRACTS, Band 79, 1973, Seite 410, Zusammenfassung Nr. 91886p, Columbus, Ohio, US; S.S. KUKALENKO et al.: "Chemistry of organic pesticides.III. Synthesis of 4-phthalimidobutanoic acids", & ZH. ORG. KHIM. 1973, 9(7), 1401-4

## Beschreibung

Die vorliegende Erfindung betrifft Persalzaktivatoren und deren Salze, die sich von Imidocarbonsäuren und Sulfimidocarbonsäuren ableiten.

Die vorliegende Erfindung betrifft auch ein Verfahren zur Herstellung dieser Persalzaktivatoren und deren Salze und ihre Verwendung.

Anorganische Persalze sind seit langem als Bleichzusätze in Waschmitteln bekannt. Als anorganische Persalze werden Verbindungen bezeichnet, die in wäßriger Lösung Wasserstoffperoxid freisetzen. Gebräuchliche anorganische Persalze umfassen Natriumperborat-Monohydrat, Natriumperborat-Tetrahydrat, Natriumpercarbonat, Natriumperoxomonophosphat, Harnstoff-Peroxohydrat, Natriumperoxid und Mischungen davon. Da sie ihre optimale Bleichkraft jedoch erst bei Temperaturen oberhalb 60°C entfalten, werden zu ihrer Aktivierung eine Reihe von organischen Verbindungen beschrieben, die während des Waschprozesses mit Wasserstoffperoxid unter Freisetzung einer Peroxicarbonsäure reagieren, welche bereits bei 40 bis 60°C bleichend wirkt. Verbindungen mit einer derartigen Wirkungsweise werden als Persalz- oder Perborataktivatoren bezeichnet.

Eine Übersicht zahlreicher bekannter Persalzaktiviatoren wie N-Acylverbindungen (Tetraacetylethylendiamin, Tetraacetylmethylendiamin, Tetraacetylglycoluril) oder aktivierte Ester (Pentaacetylglucose, Acetoxibenzolsulfonat-Natrium, Benzoyloxibenzolsulfonat-Natrium) ist z.B. in der US-Patentschrift 4 284 928 gegeben.

Daneben werden in neuerer Zeit eine Reihe organischer Peroxicarbonsäuren als Bleichsysteme für Waschmittel beschrieben. Neben bereits kommerziell erhältlichen Peroxicarbonsäuren wie Dodecandiperoxicarbonsäure (EP-A-127 782) und Monoperoxiphthalsäure (EP-A-27 693) sind Amidoperoxicarbonsäuren (EP-A-170 386) und Imidoperoxicarbonsäuren (EP-A-325 288, EP-A-349 940, EP-A-366 041 und noch nichtveröffentlichte deutsche Patentanmeldung mit dem Aktenzeichen P 4 036 647.2) beschrieben. Bei der Verwendung von Peroxicarbonsäuren in kommerziellen Bleichmitteln ergeben sich jedoch verschiedene Probleme. Bedingt durch die thermische Labilität solcher Perverbindungen neigen Bleichmittel mit einem Gehalt an Peroxicarbonsäuren dazu, während der Lagerung ihren Aktivsauerstoff zu verlieren, und man sieht sich infolge ihrer exothermen Zersetzungsreaktion vor ein Sicherheitsproblem gestellt. Zwar können diese Schwierigkeiten bis zu einem gewissen Grad durch Zusatz von Phlegmatisierungsmitteln (EP-A-376 360, EP-A-105 689, DE-A-2 737 865) und/oder eine aufwendige Granuliertechnik (EP-A-396 341, EP-A-256 443, EP-A-272 402, EP-A-200 163) kontrolliert werden, vollkommen beseitigen lassen sich die Probleme aber bislang nicht. Langkettige Peroxicarbonsäuren zeichnen sich zwar durch eine bessere Stabilität aus, besitzen aber den Nachteil, daß sie fast gänzlich wasserunlöslich sind und deshalb für Waschmittelformulierungen weniger geeignet sind.

Aus diesen Gründen besteht auch weiterhin ein dringendes Bedürfnis nach effizienten, lagerstabilen und gut wasserlöslichen Bleichmitteln auf der Basis von Persalzaktivatoren, die erst in der Waschlauge in Kombination mit einem anorganischen Persalz die bleichaktive Peroxicarbonsäure freisetzen.

Überraschenderweise wurde nun gefunden, daß die im folgenden beschriebenen Persalzaktivatoren und deren Salze, die sich von Imidocarbonsäuren und Sulfimidocarbonsäuren ableiten, eine wesentlich höhere Lagerstabilität und Wasserlöslichkeit als die bisher bekannten Persalzaktivatoren besitzen und darüber hinaus eine ausgezeichnete Bleichkraft aufweisen.

Gegenstand der vorliegenden Erfindung sind Persalzaktivatoren und deren Salze, die sich von Imidocarbonsäuren und Sulfimidocarbonsäuren, d.h. von Imidocarbonsäuren, die eine Sulfongruppe enthalten, ableiten, der allgemeinen Formel I: 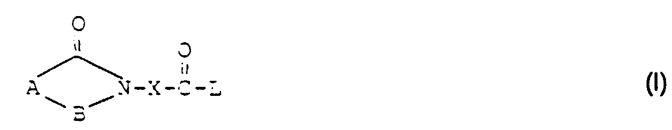 worin A eine Gruppe der Formel 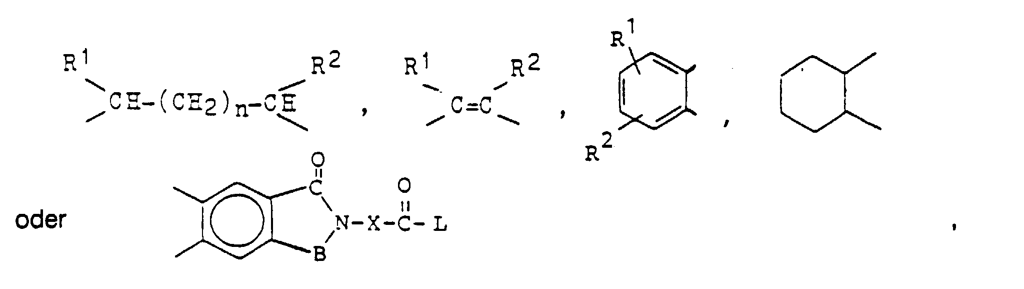
- n: die Zahlen 0, 1 oder 2,
- R¹: Wasserstoff, Chlor, Brom, C₁-C₂₀-Alkyl, C₂-C₂₀-Alkenyl, Aryl, bevorzugt Phenyl, oder Alkylaryl, bevorzugt C₁-C₄-Alkylphenyl,
- R²: Wasserstoff, Chlor, Brom oder eine Gruppe der Formel -SO₃M, -CO₂M oder -OSO₃M,
- X: C₁-C₁₉-Alkylen oder Arylen, bevorzugt Phenylen,
- B: eine Gruppe der Formeln C=O, SO₂,
- L: eine Abgangsgruppe der Formel 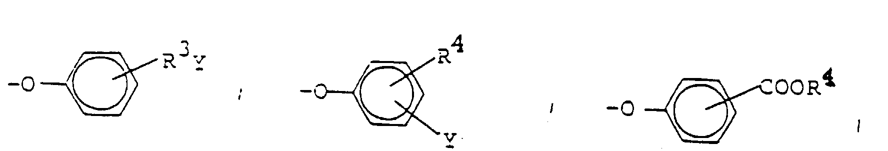 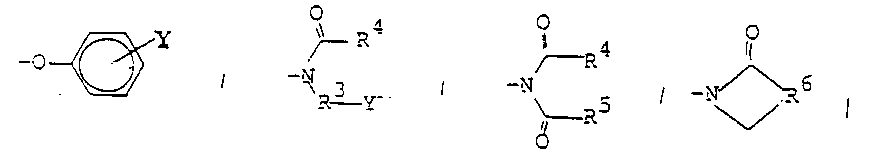 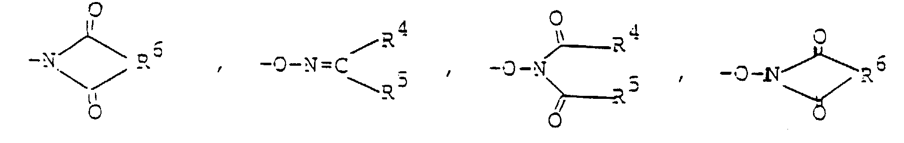
oder ein Zuckerrest,
- R³: C₁-C₁₉-Alkylen,
- R⁴ und R⁵: C₁-C₂₀-Alkyl,
- R⁶: C₁-C₁₉-Alkylen, C₂-C₂₀-Alkenylen,
- Y: Wasserstoff, Chlor, Brom oder eine Gruppe der Formel -SO₃M, -CO₂M, -OSO₃M, -CONH₂, -N(R⁷)₃Z oder -P(R⁷)₄Z,
- R⁷: C₁-C₃₀-Alkyl,
- Z: Fluorid, Chlorid, Bromid oder Jodid und
- M: Wasserstoff, ein Alkali- oder Ammonium-lon oder das Äquivalent eines Erdalkali-Ions bedeuten,
mit der Maßgabe, daß
Verbindungen der allgemeinen Formel I 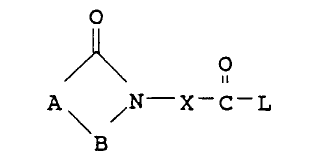 worin
- A: eine Gruppe der Formel 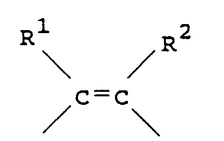
- R¹: Wasserstoff,
- R²: Wasserstoff,
- X: C₂-Alkylen,
- L: eine Abgangsgruppe der Formel 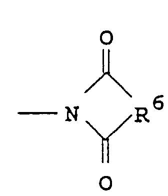 und
- R⁶: C₂-Alkylen bedeuten
und
Verbindungen der allgemeinen Formel I 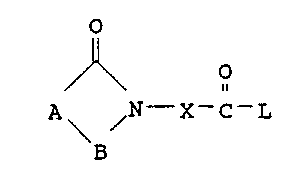 worin
- A: eine Gruppe der Formel 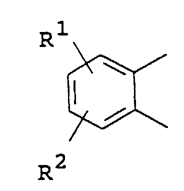
- R¹: Wasserstoff,
- R²: Wasserstoff,
- X: C₃-Alkylen,
- L: eine Abgangsgruppe der Formel 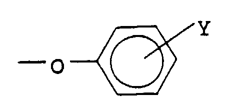 und
- Y: p-Chlor bedeuten,
ausgenommen sind. Bevorzugt sind Persalzaktivatoren und deren Salze, die sich von
Imidocarbonsäuren und Sulfimidocarbonsäuren der obigen Formel I ableiten, worin
- A: eine Gruppe der Formel -HC=CH-, 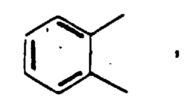 -CH₂-(CH₂)ₙ-CH₂-, 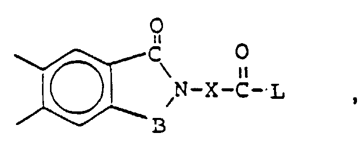 oder -CH₂-CHR¹-,
- n: die Zahlen 0 oder 1,
- R¹: C₁-C₂₀-Alkyl oder C₂-C₂₀-Alkenyl,
- X: C₄-C₈-Alkylen,
- B: eine Gruppe der Formeln C=O, SO₂,
- L: eine Abgangsgruppe der Formel

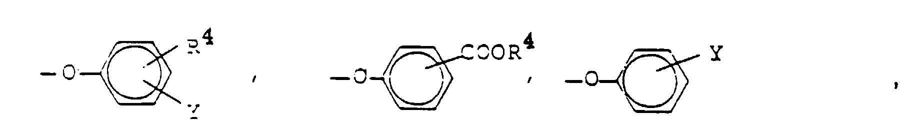

- R⁴: C₁-C₂₀-Alkyl,
- Y: Wasserstoff oder eine Gruppe der Formel -SO₃M, CO₂M, CO₂M, CONH₂, -OSO₃M, -N(R⁷)₃Z oder -P(R⁷)₄Z,
- R⁷: C₁-C₄-Alkyl, besonders bevorzugt Methyl,
- Z: Chlorid und
- M: Wasserstoff, ein Alkali- oder Ammonium-lon oder das Äquivalent eines Erdalkali-Ions bedeuten.

Insbesondere bevorzugt sind ω-Phthalimidoalkanoyloxibenzolcarbonsäuren und deren Salze, ω-Phthalimidoalkanoyloxibenzolsulfonsäuren und deren Salze, ω-2-Alkylsuccinimidoalkanoyloxibenzolcarbonsäuren und deren Salze, ω-2-Alkylsuccinimidoalkanoyloxibenzolsufonsäuren und deren Salze, ω-[1,1,3-Trioxo-3H-λ⁶-benz[α]isothiazol-2-yl]-alkanoyloxibenzolcarbonsäuren und deren Salze und ω-[1,1,3-Trioxo-3H-λ⁶-benz[α]isothiazol-2-yl]-alkanoyloxibenzolsulfonsäuren und deren Salze.

Ebenfalls Gegenstand der Erfindung ist ein Verfahren zur Herstellung der erfindungsgemäßen Persalzaktivatoren und deren Salze sowie ihre Verwendung als Bleich-, Oxidations- und Desinfektionsmittel.

Die Herstellung der Persalzaktivatoren und deren Salze der Formel I, die sich von Imidocarbonsäuren ableiten, erfolgt durch folgende Schritte:
-a- Synthese der Imidocarbonsäure
-b- Synthese der Persalzaktivatoren und deren Salze

Im folgenden werden die einzelnen Schritte näher erläutert.
Die Herstellung der Imidocarbonsäuren der allgemeinen Formel 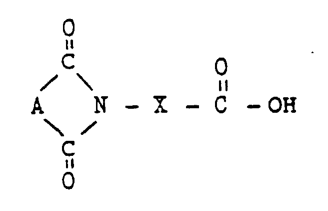 in Schritt -a- kann in an sich bekannter Weise, wie schon in der Patentanmeldung EP-A-349 940 beschrieben, durch Umsetzung von Anhydriden der Formel 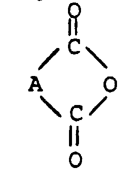 mit Aminosäuren der Formel

H₂N - X - COOH

erfolgen (s. Houben Weyl, Methoden der Organischen Chemie, XI/2, S. 17).

Als Anhydride können insbesondere Bernsteinsäureanhydrid, Glutarsäureanhydrid, Maleinsäureanhydrid, Phthalsäureanhydrid, Pyromellithsäureanhydrid, Alkyl-oder Alkenylbernsteinsäureanhydrid eingesetzt werden, als Aminosäuren ω-Aminobuttersäure, ω-Aminovaleriansäure, ω-Aminocapronsäure und ω-ω-Aminolaurinsäure.

Die von der ω-Aminobuttersäure, ω-Aminocapronsäure und ω-Aminolaurinsäure abgeleiteten Imidocarbonsäuren können besonders kostengünstig auch aus Pyrrolidon, ε-Caprolactam bzw. Laurinlactam hergestellt werden. Hierzu wird das Lactam mit dem Anhydrid unter Zugabe einer katalytischen Menge Wasser 2 bis 80 h, vorzugsweise 5 bis 25 h, bei einer Temperatur von 100 bis 280°C, vorzugsweise 120 bis 220°C, unter Inertgasatmosphäre in einem geeigneten Reaktionsgefäß eingesetzt. Der Überdruck kann 1 bis 50 bar, vorzugweise 2 bis 10 bar, betragen.

Die Herstellung der Persalzaktivatoren und deren Salze der Formel I, die sich von Imidocarbonsäuren ableiten, im Schritt -b- kann grundsätzlich nach zwei verschiedenen Syntheseverfahren erfolgen:
- das Anhydrid-Verfahren
- das Säurehalogenid-Verfahren
In dem einstufigen Anhydrid-Verfahren werden die erfindungsgemäßen Persalzaktivatoren in einem Eintopfverfahren erhalten, bei dem die Imidocarbonsäure gleichzeitig mit einem kurzkettigen Carbonsäureanhydrid und einem substituierten Hydroxybenzolderivat zur Reaktion gebracht wird. Die eingesetzten Hydroxybenzolderivate werden durch diese Umsetzung zu den Abgangsgruppen L der Persalzaktivatoren.
Dabei kann lösemittelfrei oder, wie schon in EP-A-262 895 beschrieben, in einem organischen Lösemittel gearbeitet werden. Als organische Lösemittel kann man insbesondere, aber nicht ausschließlich, hochsiedene Kohlenwasserstoffe, wie z.B. Xylol, Toluol, Oktan, Dekan oder Dodekan verwenden. Bevorzugt wird allerdings die lösemittelfreie Umsetzung, da die eingesetzten kurzkettigen Carbonsäureanhydride, wie Essigsäure-, Propionsäure- und Buttersäureanhydrid auch als Lösemittel fungieren können. Aufgrund seines günstigen Preises und seiner guten Verfügbarkeit wird Essigsäureanhydrid bevorzugt und steht der Einfachheithalber in der folgenden Beschreibung der Reaktionsbedingungen stellvertretend für alle einsetzbaren kurzkettigen Carbonsäureanhydride.

Als substituierte Hydroxybenzolderivate werden o,p-Hydroxybenzolsulfonsäuren und deren Salze oder o,p-Hydroxybenzolcarbonsäuren und deren Salze, bevorzugt p-Hydroxybenzolsulfonsäuren und deren Salze und p-Hydroxybenzolcarbonsäuren und deren Salze eingesetzt.
Zur Durchführung der Reaktion setzt man ein Gemisch aus dem substituierten Hydroxybenzolderivat, Acetanhydrid und einer Imidocarbonsäure der angegebenen Formel im molaren Verhältnis 1:1-5:1-5, bevorzugt 1:1-3:1-3, um. Zur Beschleunigung der Reaktion kann als Katalysator ein Alkali- oder Erdalkalisalz einer Carbonsäure, beispielsweise Natriumacetat, zugegeben werden. Es ist auch möglich, zunächst in einem separaten Schritt das Acetoxybenzolderivat herzustellen und dieses Produkt dann mit 1 bis 5 Mol, vorzugsweise 1 bis 3 Mol, der angegebenen Imidocarbonsäure umzuestern.
In Fällen, in denen keine genügende Durchmischung der Komponenten erreicht wird, kann es vorteilhaft sein, die Reaktion in einem beheizbaren Kneter, beispielsweise einem Sigma-Kneter, durchzuführen.

Die Reaktionstemperatur muß so hoch sein, daß die im Verlaufe der Reaktion gebildete Essigsäure abdestillieren kann. Dies ist im allgemeinen bei Temperaturen von 120-300°C, bevorzugt bei 150-250°C der Fall. Die Reaktionsdauer richtet sich nach der Art der Carbonsäure und des Katalysators.

Nach Beendigung der Reaktion läßt man das Reaktionsgemisch abkühlen und entfernt die überschüssige Carbonsäure sowie Restmengen an Acetoxibenzolsulfonat, die nicht umgeestert worden sind, durch Waschen mit einem Lösemittel. Die so erzielbaren Produktreinheiten sind gut, können aber durch häufiges Waschen mit einem geeigneten Lösemittel oder Umkristallisation noch gesteigert werden. Die dabei anfallenden Lösungen enthalten Restmengen an wieder einsetzbaren Wertstoffen und können zur Ausbeuteerhöhung in der darauf folgenden Reaktion erneut umgesetzt werden. Die gesamte Reaktion kann somit auch nach bekannten Methoden kontinuierlich ausgestaltet sein.
Darstellungsmethoden dieser Art sind beispielsweise in EP-A-105 672, EP-A-105 673 und DE-A-3 824 901 aufgeführt. Weitere Syntheseverfahren sind beschrieben in EP-A-202 698, EP-A-210 674, EP-A-140 251, EP-A-163 224, EP-A-163 225, EP-A-125 641, EP-A-165 480, EP-A-211 045, EP-A-120 591, EP-A-166 571, EP-A-204 116, EP-A-153-222, EP-A-153 223, EP-A-164 786, EP-A-168 876, EP-A-201 222, EP-A-227 194, EP-A-207 445, EP-A-220 656 und EP-A-229 890.

In dem zweistufigen Säurehalogenid-Verfahren werden die Imidocarbonsäuren in bekannter Weise in die entsprechenden Säurehalogenide, bevorzugt Säurechloride überführt (Houben-Weyl, Methoden der Organischen Chemie, Band E5, S. 593-600). In einem zweiten Reaktionsschritt werden die Imidocarbonsäurehalogenide mit den substituierten Hydroxybenzolderivaten zum erfindungsgemäßen Persalzaktivator umgesetzt. Dabei werden die Imidocarbonsäurehalogenide zusammen mit den substituierten Hydroxybenzolderivaten im molaren Verhältnis 0,1-2,5:1, bevorzugt 0,5-1,5:1, in einem inerten hochsiedenen Lösemittel, beispielsweise Toluol oder Xylol, bei Temperaturen zwischen 80 und 200°C, bevorzugt 100 bis 150°C, zur Reaktion gebracht, bis keine Gasentwicklung mehr beobachtet werden kann. Die Reaktionsdauer beträgt in der Regel zwischen 60 und 360 min. kann aber länger sein und ist abhängig von der Reaktivität des Säurehalogenids.

Nach dem Abkühlen der Reaktionsmischung wird das Lösemittel abgesaugt und der Filterkuchen gewaschen und/oder aus einen geeigneten Lösemittel umkristallisiert.

Analoge Reaktionen sind beschrieben in den Patentanmeldungen EP-A- 98 129, EP-A-148 148, EP-A-164 786 und EP-A-220 826.

Die Herstellung der Persalzaktivatoren und deren Salze der Formel I, die sich von Sulfimidocarbonsäuren ableiten, gelingt, analog der bereits skizzierten Darstellungsmethoden, durch folgende Schritte:
-c- Synthese der Sulfimidocarbonsäure
-d- Synthese der Persalzaktivatoren und deren Salze

Im folgenden werden die Schritte näher erläutert. Die Herstellung der Sulfimidocarbonsäuren der Formel 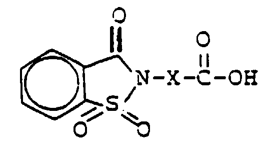 die als Saccharincarbonsäuren bezeichnet werden, im Schritt -c- kann in an sich bekannter Weise durch Umsetzung des 2-Sulfobenzoesäureanhydrids 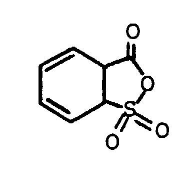 mit Aminosäuren der Formel

H₂N-X-COOH

(US-A-2 462 835) erfolgen.
Die gewünschten Sulfimidocarbonsäuren lassen sich auch durch säure- oder basenkatalysierte Hydrolyse von Saccharincarbonsäureestern (Houben-Weyl, Methoden der Organischen Chemie, E5, S. 223) gewinnen, die man aus der Reaktion von Saccharin-Natriumsalz (US-A-1,601,505, US-A-2,667,503) mit Halogencarbonsäureestern Hal-X-COOR⁸, wobei Hal Halogen und R⁸ C₁-C₅-Alkyl bedeuten, in Dimethylformamid erhält (J. Org. Chem. 21 (1956) 583; noch nicht veröffentlichte deutsche Patentanmeldung mit dem Aktenzeichen P 4 036 647.2). Besonders geeignet zur Herstellung der erfindungsgemäßen Persalzaktivatoren sind Saccharincarbonsäuren, die sich in ihrer Alkylkette unterscheiden, wie z.B. 3-[1,1,3-Trioxo-3H-λ⁶-benz[α]-isothiazol-2-yl]-propansäure, 4-[1,1,3-Trioxo-2H-λ⁶-benz[α]-isothiazol-2-yl]-butansäure und 6-[1,1,3-Trioxo-3H-λ⁶-benz[α]-isothiazol-2-yl]-hexansäure.

Zur Herstellung der Persalzaktivatoren und deren Salze der Formel I im Schritt - d-, die sich von Sulfimidocarbonsäuren ableiten, können ebenfalls zwei verschiedene Synthesewege beschritten werden:
- das Anhydrid-Verfahren
- das Säurehalogenid-Verfahren

In dem einstufigen Anhydrid-Verfahren werden die erfindungsgemäßen Persalzaktivatoren in einem Eintopfverfahren erhalten, bei dem die Sulfimidocarbonsäuren analog dem für Imidocarbonsäuren beschriebenen Verfahren umgesetzt werden.
In dem zweistufigen Säurehalogenid-Verfahren werden die Sulfimidocarbonsäuren in bekannter Weise in die entsprechenden Säurehalogenide, bevorzugt Säurechloride, überführt.
Dabei ist auch eine direkte Umwandlung von Saccharincarbonsäureester in die entsprechende Saccharincarbonsäurehalogenide nach literaturbekannten Methoden möglich (Houben-Weyl, Methoden der Organischen Chemie, E5, S. 604).
In einem zweiten Reaktionsschritt werden die Sulfimidocarbonsäurehalogenide dann analog dem für Imidocarbonsäuren beschriebenen Verfahren weiter umgesetzt.

Bei Persalzaktivatoren mit Abgangsgruppen der Formel 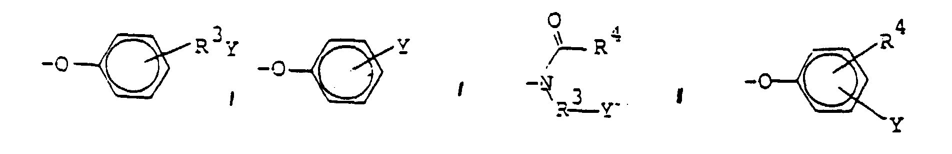 kann Y ein substituiertes Ammoniumion -N(R⁷)₃Z oder ein substituiertes Phosphoniumion -P(R⁷)₄Z sein. Sowohl bei -N(R⁷)₃Z als auch bei -P(R⁷)₄Z ist R⁷ C₁-C₃₀-Alkyl und Z ein negativ geladenes Gegenion. Im Falle von -N(R⁷)₃Z sind zwei der Reste R⁷ bevorzugt C₁-C₄-Alkyl, besonders bevorzugt Methyl und einer der Reste R⁷ eine längerkettige Alkylgruppe z.B. C₈-C₃₀-Alkyl.
Im Falle von -P(R⁷)₄Z sind drei der Reste R⁷ bevorzugt C₁-C₄-Alkyl, besonders bevorzugt Methyl und einer der Reste R⁷ eine längerkettige Alkylgruppe z.B. C₈-C₃₀-Alkyl. Sowohl bei -N(R⁷)₃Z als auch bei -P(R⁷)₄Z können R⁷ gleich oder verschieden sein.

Persalzaktivatoren mit Abgangsgruppen der Formel 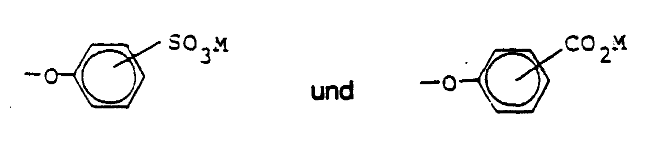 sind hinsichtlich Herstellung, Preis und Wasserlöslichkeit von besonderem Interesse. Eine Übersicht der veröffentlichten Darstellungsmethoden findet sich in den Patentanmeldungen EP-A-373 743 und JP-A-2 182-795.

Die erfindungsgemäßen Persalzaktivatoren und deren Salze sind fest, nahezu geruchlos, besitzen einen niedrigen Dampfdruck und sind von ausgezeichneter thermischer Stabilität. In Kombination mit einem anorganischen Persalz können sie zu Bleich-, Oxidations- oder Desinfektionszwecken eingesetzt werden. Anorganische Persalze wie Natriumperborat-Monohydrat, Natriumperborat-Tetrahydrat und Natriumpercarbonat werden für die erfindungsgemäßen Aktivatoren als anorganisches Persalz in Wasch- und Bleichmittelformulierungen bevorzugt.

Bevorzugt werden sie als Bleichmittel in festen oder flüssigen Wasch-und Reinigungsmitteln eingesetzt, da ihre bleichende und desinfizierende Wirkung bereits in einem weiten Temperaturbereich unterhalb von 60°C voll wirksam wird.

Zur Einarbeitung in pulverförmige Waschmittel eignen sich insbesondere die erfindungsgemäßen Persalzaktivatoren oder deren Salze in granulierter, extrudierter, tablettierter oder agglomerierter Form. In basischen Waschmittelformulierungen werden die erfindungsgemäßen Persalzaktivatoren oder deren Salze bevorzugt in granulierter Form eingesetzt. Als Granulierhilfsmittel eignen sich organische Fettsäuren, Alkoholethylate, Carboxymethylcellulose oder filmbildende Polymere wie Polyacrylsäuren. Eine Kombination der Persalzaktivatoren mit einem Persalz in einem Granulat ist möglich. In diesem Falle beträgt das Verhältnis von Persalz zu Persalzaktivator 1:10 bis 10:1, vorzugsweise 1:3 bis 3:1.

Im Wasch- oder Reinigungsmittel ist eine Kombination mit anderen Perborataktivatoren wie z.B. Tetraacetylethylendiamin oder Nonanoyloxibenzolsulfonat-Natrium oder organsichen Peroxicarbonsäuren wie Phthalimidoperoxihexansäure oder Dodecandiperoxidisäure möglich.

Als weitere Zusätze können die Waschmittelformulierungen enthalten: anionische, nichtionische, kationische oder zwitterionische Tenside, anorganische Builder wie Zeolith oder Schichtsilikate, Cobuilder wie Polycarboxilate oder organische Builder wie Citronensäure. Daneben sind möglich optische Aufheller, Enzyme und Parfümstoffe.

Sehr gute Bleichergebnisse werden im pH-Bereich zwischen 8 und 9 erzielt. Eine Veränderung des pH-Wertes der Waschlauge während des Waschprozesses kann durch die Zugabe von Protonenspendern (organische oder anorganische Säuren, Ester oder Anhydride) während des Waschprozesses erfolgen.

Unter dem Begriff WS-Gehalt (WS steht für Wirksubstanz) ist in den nachfolgenden Beispielen der Anteil an Wirksubstanz im Produkt zu verstehen, der durch 2-Phasen-Titration nach Epton (Nature 160, 756 [1947]) bestimmt wird.

Die Herstellung der erfindungsgemäßen Persalzaktivatoren wird durch die folgenden Beispiele erläutert:

### Beispiel 1

### Natrium-α-phthalimidoacetoxibenzolsulfonat

98.0 g (0.5 Mol) wasserfreies Natriumphenolsulfonat und 112.0 g (0,5 Mol) α-Phthalimidoessigsäurechlorid werden mit 500 g Xylol versetzt und 15 h bei 140°C gehalten. Nach dem Abkühlen wird die Reaktionsmischung in Aceton aufgenommen, die Lösemittel über einen Büchnertrichter abgesaugt und der Kristallbrei noch zweimal mit je 80 ml Aceton gewaschen. Nach der Umkristallisation aus 90 %igem Ethanol trocknet man das erhaltene Produkt bei 40°C im Wasserstrahlvakuum.
Ausbeute: 168 g (88 %), Fp. > 220°C
WS-Gehalt: 93 % (Epton-Titration)

### Beispiel 2

### Natrium-γ-phthalimidobutanoyloxibenzolsulfonat

148.0 g (755 mmol) wasserfreies Natriumphenolsulfonat und 190.0 g (755 mmol) γ-Phthalimidobutansäurechlorid werden in 200 g Xylol für 4 h bei 140°C zur Reaktion gebracht und wie in Beispiel 1 aufgearbeitet. Das Rohprodukt kristallisiert man aus Methanol um und trocknet das weiß-kristalline Produkt bei 40°C im Wasserstrahlvakuum.
Ausbeute: 280 g (90 %), Fp. > 220°C
WS-Gehalt: 92 % (Epton-Titration)

### Beispiel 3

### Natrium-ε-phthalimidohexanoyloxybenzolsulfonat

118.0 g (600 mol) wasserfreies Natriumbenzolsulfonat und 170.0 g (600 mmol) ε-Phthalimidohexansäurechlorid werden in 100 g Xylol für 1 h bei 125°C zur Reaktion gebracht und wie in Beispiel 1 beschrieben aufgearbeitet. Nach der Umkristallisation aus Ethanol trocknet man das weiße Produkt bei 40°C im Wasserstrahlvakuum.
¹H-NMR (D₂O, 100 MHz): δ = 1.15-1.83 (m, 6H), 2.5 (t, 2H), 3.6 (t, 2H), 7.0 (m, 2H) 7.5-7.8 (m, 6H).
Ausbeute: 250 g (95 %), Fp. > 220°C
WS-Gehalt: 97 % (Epton-Titration)

### Beispiel 4

### Natrium-ω-phthalimidododekanoyloxibenzolsulfonat

78.5 g (400 mmol) wasserfreies Natriumbenzolsulfonat und 145.6 g (400 mmol) w-Phthalimidododekansaurechlorid werden in 200 g Xylol für 4 h bei 130-140°C zur Reaktion gebracht und wie in Beispiel 1 beschrieben aufgearbeitet. Das aus 90 %igem Ethanol umkristallisierte Produkt trocknet man anschießend bei 40°C im Wasserstrahlvakuum.
Ausbeute: 178 g (85 %), Fp. > 220°C
WS-Gehalt = 98 % (Epton-Titration)

### Beispiel 5

### Natrium-ω-(2-dodecylsuccinimido)-acetoxibenzolsulfonat

78.5 g (400 mmol) wasserfreies Natriumphenolsulfonat und 137.4 g (400 mmol) ω-(2-Dodecylsuccinimido)-essigsäurechlorid werden in 200 g Xylol für 25 h bei 140°C zur Reaktion gebracht und wie in Beispiel 1 beschrieben aufgearbeitet. Das aus 90 %igem Ethanol umkristallisierte Produkt trocknet man anschließend bei 40°C im Wasserstrahlvakuum.
Ausbeute: 145 g (72 %), Fp. > 220°C
WS-Gehalt: 85 % (Epton-Titration)

### Beispiel 6

### Natrium-ω-trimellithimidohexanoyloxibenzolsulfonat

39.3 g (200 mmol) wasserfreies Natriumbenzolsulfonat und 64.7 g (200 mmol) ω-Trimellithimidohexansäurechlorid werden in 200 g Xylol für 4 h bei 140°C zur Reaktion gebracht und wie in Beispiel 1 beschrieben aufgearbeitet. Das aus 90 %igem Ethanol umkristallisierte Produkt wird anschließend bei 40°C im Wasserstrahlvakuum getrocknet.
Ausbeute: 78 g (80 %), Fp. > 220°C
WS-Gehalt: 91 % (Epton-Titration)

### Beispiel 7

### Natrium-ε-phthalimidohexanoyloxibenzolsulfonat

261.0 g (1,0 Mol) ε-Phthalimidohexansäure, 98.0 g (0,5 Mol) wasserfreies Natriumphenolsulfonat, 61.0 g (0,6 Mol) Acetanhydrid und 2 g Natriumacetat werden zusammen für 2,5 h auf 150°C erhitzt, wobei die Reaktionsmischung bereits nach kurzer Zeit eine hochviskose Konsistenz annimmt und schwer rührbar wird. Danach wird die Temperatur langsam auf 210°C gesteigert und die gebildete Essigsäure unter Zuhilfenahme eines über die Reaktionsmischung geführten Stickstoffstromes abdestilliert. Die jetzt wieder dünnflüssige Mischung hält man anschließend noch für 1 h bei 200°C. Um die überschüssige Phthalimidohexansäure herauszulösen, wird die noch rührbare Reaktionsmischung bei etwa 80°C unter guter Kühlung mit 400 ml Aceton versetzt und danach noch zweimal mit je 200 ml Aceton bei 60°C ausgerührt. Zur weiteren Reinigung kann der Rückstand zusätzlich mit Ethanol gewaschen werden, so daß das während der Reaktion gebildete Acetoxibenzolsulfonat ebenfalls herausgelöst wird. Alternativ zur beschriebenen Aufarbeitung kann man auch die heiße, dünnflüssige Reaktionsschmelze zum Abkühlen auf ein Blech gießen und diese nach dem Erstarren pulverisieren. Danach wird das Rohprodukt mit Aceton und Ethanol gereinigt.
Ausbeute: 115 g (88 %, bezogen auf Natriumphenolsulfonat)
WS-Gehalt: 85 % (Epton-Titration)

### Ausprüfung von Perborataktivatoren auf Basis von Imidoperoxicarbonsäuren

### Beispiel 8

### Waschversuche im Launder-O-Meter

Die Waschversuche wurden in einem Launder-O-Meter unter folgenden Bedingungen durchgeführt:

| | |
|---|---|
| Wasserhärte | 15°dH |
| Waschtemperaturen | 20, 40, 60°C |
| Waschzeit | 15 min |
| Waschmittel | Mischung aus 1,5 g/l WMP-Testwaschmittel (WFK) und 0,9 g/l Natriumperborat-Monohydrat |
| Bleichtestgewebe | Tee auf Baumwolle (WFK) |
| | Kaffee auf Baumwolle (WFK) |
| | Rotwein auf Baumwolle (EMPA) |
| EMPA: Eidgenössische Materialprüfanstalt, St. Gallen WFK: Wäschereiforschung Krefeld | |

Die zugesetzten Perborataktivatoren wurden so zudosiert, daß nach erfolgter Perhydrolyse jeweils 25 mg Aktivsauerstoff in Form der entsprechenden Persäure vorlagen.

Als Perborataktivatoren wurden eingesetzt:
PAPA: Phthalimidohexanoyloxibenzolsulfonat-Natrium (erfindungsgemäß) NOBS: Nonanoyloxibenzolsulfonat-Natrium

Nach Beendigung des Waschprozesses wurde der Weißgrad des Gewebes mittels eines Remissions-Photometers bestimmt. Die angegebenen Remissionswerte sind gemittelt über die drei Bleichtestgewebe.

| | % Remission | | |
|---|---|---|---|
| | 20°C | 40°C | 60°C |
| WMP/PB*1 | 50,8 | 50,9 | 52,8 |
| WMP/PB*1/NOBS | 57,0 | 63,0 | 70,3 |
| WMP/PB*1/PAPA | 58,6 | 64,0 | 71,0 |
| PB*1: Perboratmonohydrat-Natriumsalz | | | |

### Beispiel 9

### Waschversuche im Launder-O-Meter

Die Versuche wurden analog Beispiel 8 mit folgenden Änderungen durchgeführt:

| | |
|---|---|
| Waschmittel | Mischung aus 1,5 g/l phosphathaltigem IEC-Waschmittel (WFK) und 0,9 g/l Perborat Tetrahydrat |
| Bleichtestgewebe | Tee auf Baumwolle (WFK) |
| | Rotwein auf Baumwolle (EMPA) |

Perborataktivatoren:

| | |
|---|---|
| A1 | Phthalimidobutanoyloxibenzolsufonat-Na |
| A2 | Trimellithimidohexanoyloxibenzolsulfonat-Na |
| A3 | Phthalimidoacetoyloxibenzolsulfonat-Na |
| TAED | Tetraacetylethylendiamin (Vergleich) |

| % Remission | | | | |
|---|---|---|---|---|
| | Tee | | Rotwein | |
| | 20°C | 40°C | 20°C | 40°C |
| A1: | 52,7 | 57,1 | 62,1 | 65,2 |
| A2: | 47,9 | 51,9 | 56,9 | 61,2 |
| A3: | 48,2 | 52,3 | 57,7 | 61,4 |
| TAED | 47,2 | 51,3 | 56,6 | 60,8 |

### Beispiel 10

### Waschversuche im Launder-O-Meter

Es wurden Waschversuche im Launder-O-Meter unter folgenden Bedingungen durchgeführt:

| | |
|---|---|
| Wasserhärte | 5,6°dH |
| Waschtemperaturen | 25, 40, 55°C |
| Waschzeit | 15 min |
| Waschmittel | 2 g/l Tide® (incl. Bleichsystem) |
| Bleichsystem | 7,5 % Natriumperborat Monohydrat und 5 % Perborataktivator (s. Beispiel 8) |
| Bleichtestgewebe | Tee auf Baumwolle (WFK) |
| | Rotwein auf Baumwolle (WFK) |
| | Rotwein auf Baumwolle (EMPA) |
| (Tide®: Waschmittel, Hersteller: Procter u. Gamble) | |

| | % Remission*) | | |
|---|---|---|---|
| | 25°C | 40°C | 55°C |
| Tide®/PB*1 | 53,3 | 54,2 | 55,2 |
| Tide®/PB*1/NOBS | 55,0 | 56,1 | 57,8 |
| Tide®/PB*1/PAPA | 55,4 | 56,1 | 59,1 |

| | | | |
|---|---|---|---|
| *) gemittelt über die drei Bleichtestgewebe | | | |

### Beispiel 11

### Mehrfachwäsche im Launder-O-Meter

Es wurden Waschversuche im Launder-O-Meter unter folgenden Bedingungen durchgeführt:

| | |
|---|---|
| Wasserhärte | 5,6°dH |
| Waschtemperatur | 40°C |
| Waschzeit | 15 min |
| Waschcyclen | 4 |
| Waschmittel | 1,5 g/l Tide® |
| Persalz | 0,9 g/l Natriumperborat-Monohydrat |

Perborataktivatoren

| | |
|---|---|
| PAPA | Phthalimidohexanoyloxibenzolsulfonat-Natrium (erfindungsgemäß) |
| NOBS | Nonanoyloxibenzolsulfonat-Natrium (Vergleich) |
| TAED | Tetraacetylethylendiamin (Vergleich) |
| Bleichtestgewebe | Tee auf Baumwolle (WFK) |
| | Rotwein auf Baumwolle (EMPA) |
| | Remazol Brillant Rot GG® (Textilfarbstoff (Hoechst AG)) auf Baumwolle |

Die Perborataktivatoren wurden so dosiert, daß nach erfolgter Perhydrolyse jeweils 3 mg/l Aktivsauerstoff in Form der entsprechenden Persäure in der Waschlauge vorlagen.

| | % Remission | |
|---|---|---|
| | ⌀ Tee/Rotwein | Remazol Brillant Rot |
| Tide® | 47,9 | 26,8 |
| Tide®/PB*1/TAED | 59,9 | 28,3 |
| Tide®/PB*1/NOBS | 61,4 | 29,5 |
| Tide®/PB*1/PAPA | 61,4 | 28,4 |

Die Ergebnisse zeigen, daß der erfindungsgemäße Perborataktivator PAPA gute Bleichleistung zeigt, ohne Farbschädigung hervorzurufen.

### Beispiel 12

### Waschversuche mit variablem pH-Wert der Waschflotte

Es wurden Waschversuche im Becherglas unter folgenden Bedingungen durchgeführt:

| | |
|---|---|
| Wasserhärte | 5,6°dH |
| Waschtemperatur | 22°C |
| Waschzeit | 15 min |
| Waschmittel | 1,75 g/l Tide® |
| Bleichsystem | 0,1 g/l Perborataktivator |
| | 0,15 g/l Natriumperborat Monohydrat |
| Bleichtestgewebe | Rotwein auf Baumwolle (EMPA) |
| AnfangspH-Wert der Waschlauge | 10,3 |

5 min nach Beginn des Waschprozesses wurde der pH-Wert der Waschlauge mit H₂SO₄ auf den gewünschten pH-Wert eingestellt.

| | % Remission | | |
|---|---|---|---|
| | pH 10,3 | pH 9 | pH8 |
| Tide® | 49,1 | 49,0 | 49,3 |
| Tide®/PB*1/TAED | 50,6 | 51,9 | 50,7 |
| Tide®/PB*1/PAPA | 51,2 | 53,0 | 51,9 |

Die Ergebnisse zeigen, daß das Bleichoptimum des erfindungsgemäßen Perborataktivators im pH-Bereich zwischen 8 und 9 liegt.

## Patentansprüche (Patentansprüche für folgende(n) Vertragsstaat(en): AT, BE, CH, DE, FR, GB, IT, LI, NL)

1. Persalzaktivatoren und deren Salze, die sich von Imidocarbonsäuren und Sulfimidocarbonsäuren ableiten, der allgemeinen Formel I: 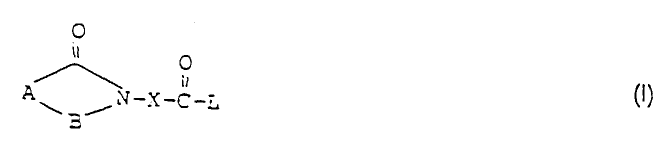 worin A eine Gruppe der Formel 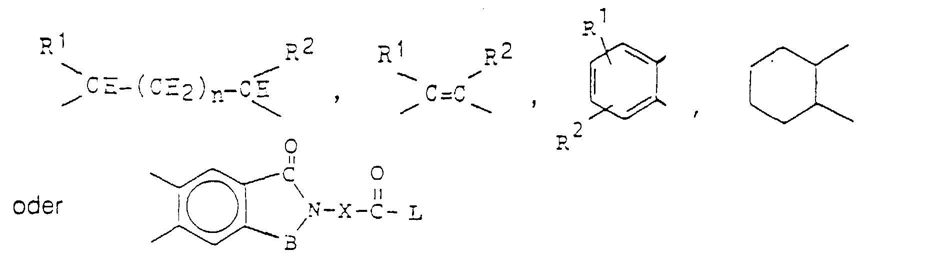
n die Zahlen 0, 1 oder 2,
R¹ Wasserstoff, Chlor, Brom, C₁-C₂₀-Alkyl, C₂-C₂₀-Alkenyl, Aryl, bevorzugt Phenyl, oder Alkylaryl, bevorzugt C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Arylen, bevorzugt Phenylen,
B eine Gruppe der Formeln C=O, SO₂,
L eine Abgangsgruppe der Formel 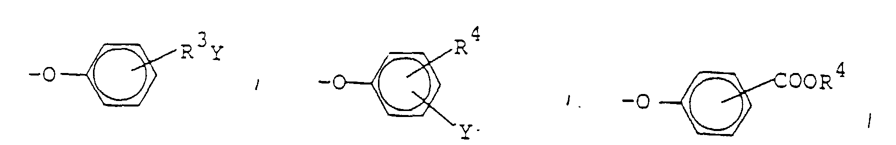 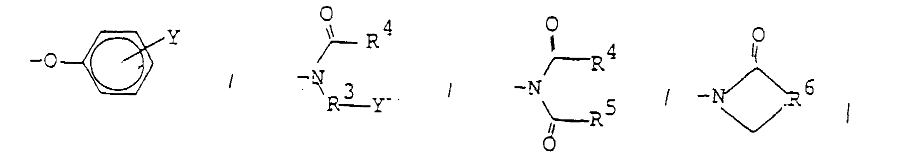 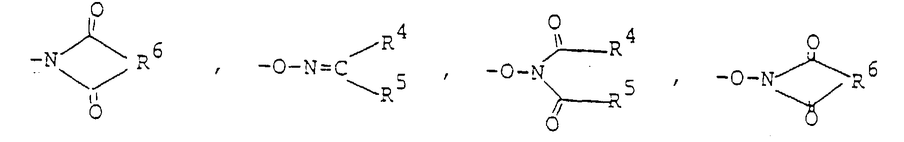
oder ein Zuckerrest,
R³ C₁-C₁₉-Alkylen,
R⁴, R⁵ C₁-C₂₀-Alkyl,
R⁶ C₁-C₁₉-Alkylen, C₂-C₂₀-Alkenylen,
Y Wasserstoff, Chlor, Brom oder eine Gruppe der Formel -SO₃M, -CO₂M, -OSO₃M, -CONH₂, -N(R⁷)₃Z oder -P(R⁷)₄Z,
R⁷ C₁-C₃₀-Alkyl,
Z Fluorid, Chlorid, Bromid oder Jodid und
M Wasserstoff, ein Alkali- oder Ammonium-lon oder das Äquivalent eines Erdalkali-Ions bedeuten.
mit der Maßgabe, daß
Verbindungen der allgemeinen Formel I 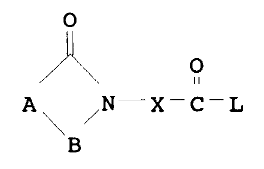 worin
A eine Gruppe der Formel 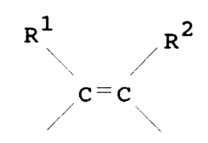
R¹ Wasserstoff,
R² Wasserstoff,
X C₂-Alkylen,
L eine Abgangsgruppe der Formel 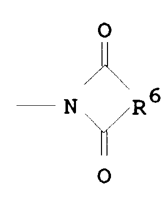 und
R⁶ C₂-Alkylen bedeuten
und
Verbindungen der allgemeinen Formel I 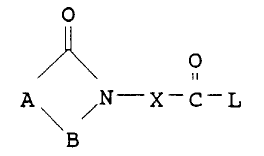 worin
A eine Gruppe der Formel 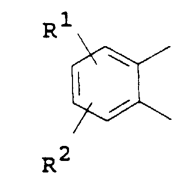
R¹ Wasserstoff,
R² Wasserstoff,
X C₃-Alkylen,
L eine Abgangsgruppe der Formel 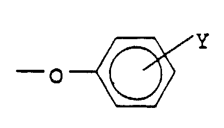 und
Y p-Chlor bedeuten,
ausgenommen sind.

2. Verbindungen nach Anspruch 1, worin
A eine Gruppe der Formel -HC=CH-, 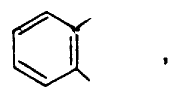 -CH₂-(CH₂)ₙ-CH₂-, 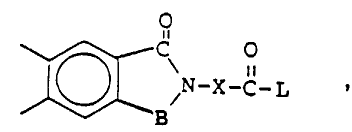 oder -CH₂-CHR¹-,
n die Zahlen 0 oder 1,
R¹ C₁-C₂₀-Alkyl oder C₂-C₂₀-Alkenyl,
X C₄-C₈-Alkylen,
B eine Gruppe der Formeln C=O, SO₂,
L eine Abgangsgruppe der Formel 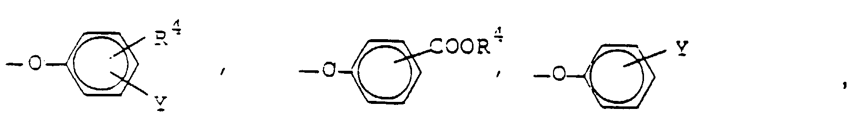
R4 Wasserstoff, Alkyl,
Y Wasserstoff oder eine Gruppe der Formel -SO₃M, -CO₂M, -OSO₃M, -CONH₂, -N(R⁷)₃Z oder -P(R⁷)₄Z,
R⁷ C₁-C₄-Alkyl, besonders bevorzugt Methyl,
Z Chlorid und
M Wasserstoff, ein Alkali- oder Ammonium-lon oder das Äquivalent eines Erdalkali-Ions bedeuten.

3. Verbindungen nach Anspruch 1 oder 2, worin
A eine Gruppe der Formel
Phenyl, -CH₂-CHR¹-,
R¹ C₁-C₂₀-Alkyl,
B eine Gruppe der Formel C=O, SO₂,
X C₄-C₈-Alkylen,
L eine Abgangsgruppe der Formel 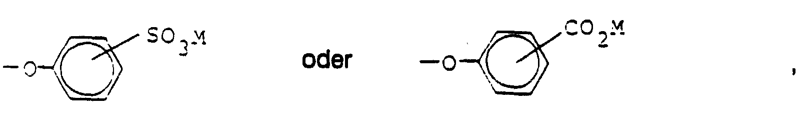 und
M Wasserstoff, ein Alkali- oder das Äquivalent eines Erdalkali-Ions bedeuten.

4. Verfahren zur Herstellung der Verbindungen nach einem der Ansprüche 1 bis 3, dadurch gekennzeichnet, daß eine Imidocarbonsäure oder Sulfimidocarbonsäure der Formel 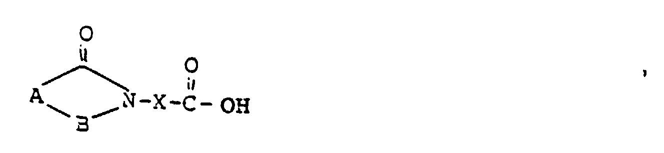 worin
A eine Gruppe der Formel 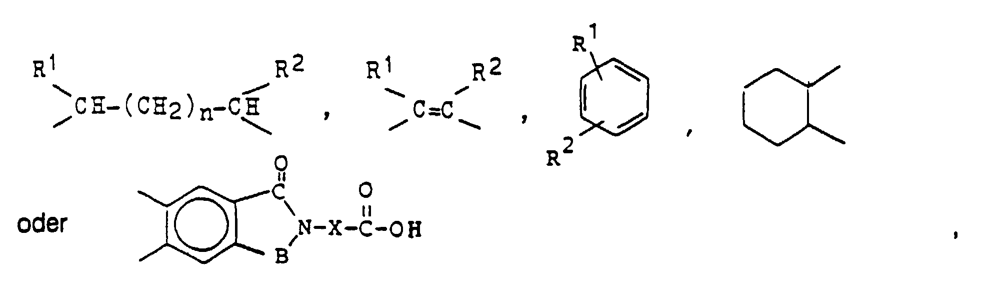
n die Zahlen 0, 1 oder 2,
R¹ Wasserstoff, Chlor, Brom, C₁-C₂₀-Alkyl, C₂-C₂₀-Alkenyl, Phenyl oder C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Phenylen
B eine Gruppe der Formeln C=O, SO₂,
bedeuten, gleichzeitig mit einem Gemisch aus einem kurzkettigen Carbonsäureanhydrid, wie Essigsäureanhydrid, Propionsäureanhydrid, Buttersäureanhydrid, bevorzugt Essigsäureanhydrid, und einem mit einer Carbonsäuregruppe oder Sulfonsäuregruppe substituierten Hydroxybenzolderivat und deren Salze in einem organischen Lösungsmittel im molaren Verhältnis 1-5:1-5:1, bevorzugt 1-3:1-3:1, umgesetzt werden.

5. Verfahren nach Anspruch 4, dadurch gekennzeichnet, daß als organische Lösungsmittel bevorzugt hochsiedende Kohlenwasserstoffe und/oder bevorzugt kurzkettige Carbonsäureanhydride verwendet werden.

6. Verfahren nach Anspruch 4 oder 5, dadurch gekennzeichnet, daß die Reaktionstemperatur zwischen 120°C und 300°C, bevorzugt zwischen 150°C und 250°C, liegt.

7. Verfahren nach einem der Ansprüche 4 bis 6, dadurch gekennzeichnet, daß als Katalysator ein Alkali- oder Erdalkalisalz einer Carbonsäure verwendet wird.

8. Verfahren zur Herstellung der Verbindungen nach einem der Ansprüche 1 bis 3, dadurch gekennzeichnet, daß ein Imidocarbonsäurehalogenid oder Sulfimidocarbonsäurehalogenid der Formel 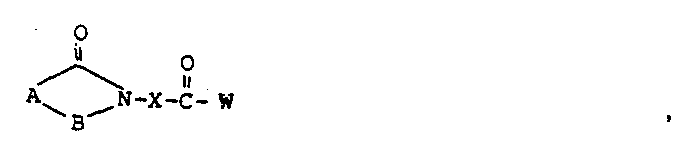 worin
A eine Gruppe der Formel 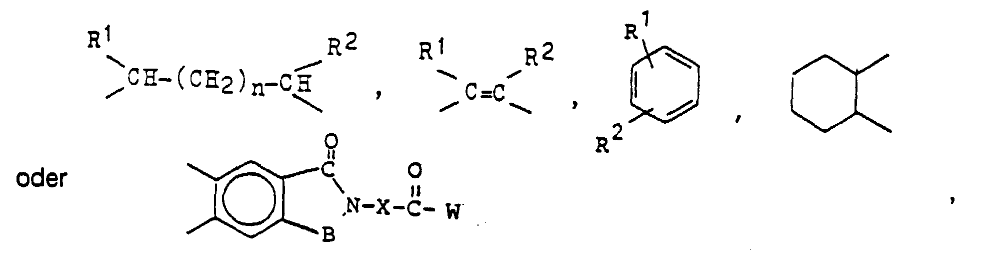
n die Zahlen 0, 1 oder 2,
R¹ Wasserstoff, Chlor, Brom, C₁-C₂₀-Alkyl, C₂-C₂₀-Alkenyl, Phenyl oder C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Phenylen,
B eine Gruppe der Formeln C=O, SO₂,
W Fluor, Chlor, Brom, Jod, bevorzugt Chlor,
bedeuten, mit einem mit einer Carbonsäuregruppe oder Sulfonsäuregruppe substituierten Hydroxybenzolderivat und deren Salze im molaren Verhältnis 0,1-2,5:1, bevorzugt 0,5-1,5:1, in einem inerten, organischen, hochsiedenden Lösungsmittel bei einer Temperatur zwischen 80°C und 200°C umgesetzt werden.

9. Verfahren nach Anspruch 8, dadurch gekennzeichnet, daß als inerte, organische Lösungsmittel bevorzugt hochsiedende Kohlenwasserstoffe verwendet werden.

10. Verfahren nach Anspruch 8 oder 9, dadurch gekennzeichnet, daß die Reaktionstemperatur zwischen 80°C und 200°C, bevorzugt zwischen 100°C und 150°C, liegt.

11. Verfahren nach einem der Ansprüche 8 bis 10, dadurch gekennzeichnet, daß die Reaktionsdauer zwischen 60 und 360 min. liegt.

12. Verwendung der Verbindungen gemäß Anspruch 1 bis 3 als Bleich-, Oxidations- und Desinfektionsmittel in Wasch- und Reinigungsmitteln.

13. Verfahren zum Bleichen von textilen Gegenständen, dadurch gekennzeichnet, daß man als Bleichmittel Verbindungen gemäß Anspruch 1 verwendet und der pH-Wert der verwendeten Waschlauge kleiner 10,3 ist, bevorzugt in einem Bereich von 8 bis 10,3 liegt.

## Patentansprüche (Patentansprüche für folgende(n) Vertragsstaat(en): ES)

1. Bleich-, Oxidations- oder Desinfektionsmittel enthaltend Persalzaktivatoren und deren Salze, die sich von Imidocarbonsäuren und Sulfimidocarbonsäuren ableiten, der allgemeinen Formel I: 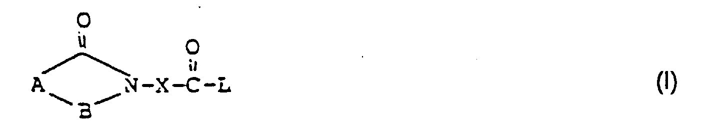 worin A eine Gruppe der Formel 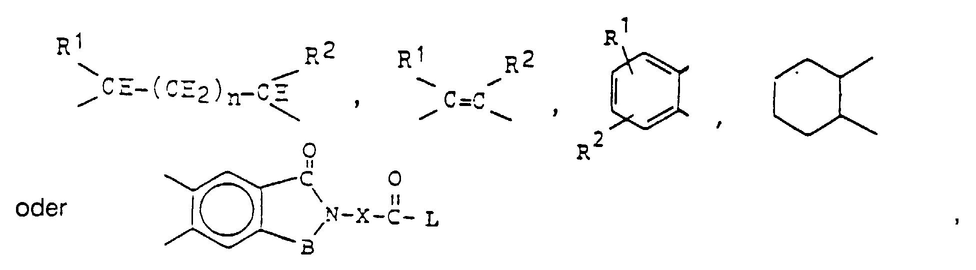
n die Zahlen 0, 1 oder 2,
R¹ Wasserstoff, Chlor, Brom, C₁-C₂₀-Alkyl, C₂-C₂₀-Alkenyl, Aryl, bevorzugt Phenyl, oder Alkylaryl, bevorzugt C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Arylen, bevorzugt Phenylen,
B eine Gruppe der Formeln C=O, SO₂,
L eine Abgangsgruppe der Formel 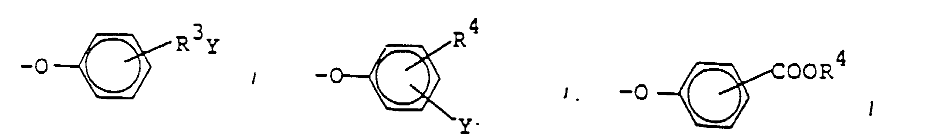 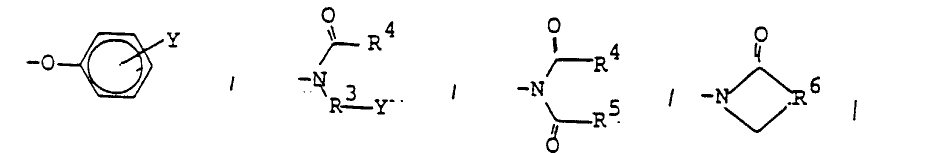 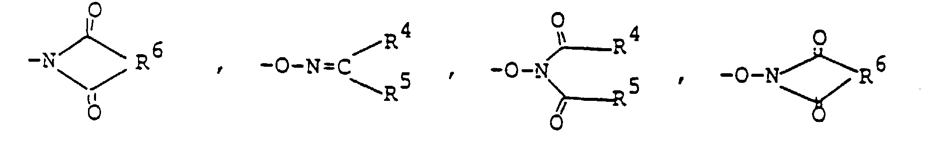 oder ein Zuckerrest,
R³ C₁-C₁₉-Alkylen,
R⁴, R⁵ C₁-C₂₀-Alkyl,
R⁶ C₁-C₁₉-Alkylen, C₂-C₂₀-Alkenylen,
Y Wasserstoff, Chlor, Brom oder eine Gruppe der Formel -SO₃M, -CO₂M, -OSO₃M, -CONH₂, -N(R⁷)₃Z oder -P(R⁷)₄Z,
R⁷ C₁-C₃₀-Alkyl,
Z Fluorid, Chlorid, Bromid oder Jodid und
M Wasserstoff, ein Alkali- oder Ammonium-lon oder das Äquivalent eines Erdalkali-Ions bedeuten,
mit der Maßgabe, daß
Verbindungen der allgemeinen Formel I 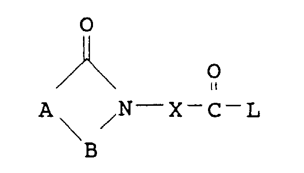 worin
A eine Gruppe der Formel 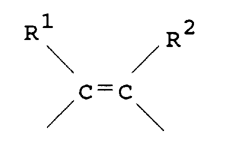
R⁷ Wasserstoff,
R² Wasserstoff,
X C₂-Alkylen,
L eine Abgangsgruppe der Formel 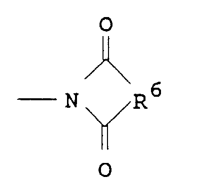 und
R⁶ C₂-Alkylen bedeuten
und
Verbindungen der allgemeinen Formel I 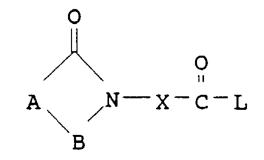 worin
A eine Gruppe der Formel 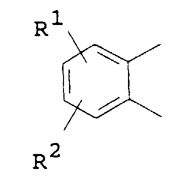
R¹ Wasserstoff,
R² Wasserstoff,
X C₃-Alkylen,
L eine Abgangsgruppe der Formel 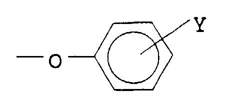 und
Y p-Chlor bedeuten,
ausgenommen sind.

2. Bleich-, Oxidations- oder Desinfektionsmittel enthaltend Verbindungen nach Anspruch 1, worin
A eine Gruppe der Formel -HC=CH-, 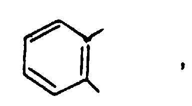 -CH₂-(CH₂)ₙ-CH₂-, 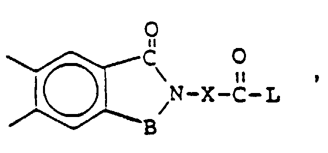 oder -CH₂-CHR¹-,
n die Zahlen 0 oder 1,
R¹ C₁-C₂₀-Alkyl oder C₂-C_{2O}-Alkenyl,
X C₄-C₈-Alkylen,
B eine Gruppe der Formeln C=O, SO₂,
L eine Abgangsgruppe der Formel 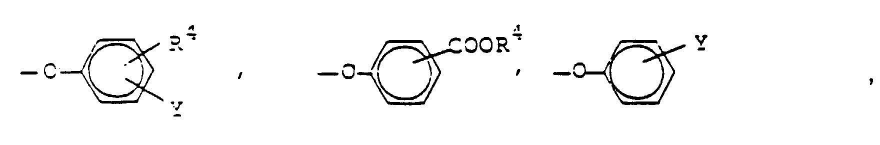
R⁴ Wasserstoff, Alkyl,
Y Wasserstoff oder eine Gruppe der Formel -SO₃M, -CO₂M, -OSO₃M, -CONH₂, -N(R⁷)₃Z oder -P(R⁷)₄Z,
R⁷ C₁-C₄-Alkyl, besonders bevorzugt Methyl,
Z Chlorid und
M Wasserstoff, ein Alkali- oder Ammonium-lon oder das Äquivalent eines Erdalkali-Ions bedeuten.

3. Bleich-, Oxidations- oder Desinfektionsmittel enthaltend Verbindungen nach Anspruch 1 oder 2, worin
A eine Gruppe der Formel
Phenyl, -CH₂-CHR¹-,
R¹ C₁-C₂₀-Alkyl,
B eine Gruppe der Formel C=O, SO₂,
X C₄-C₈-Alkylen,
L eine Abgangsgruppe der Formel 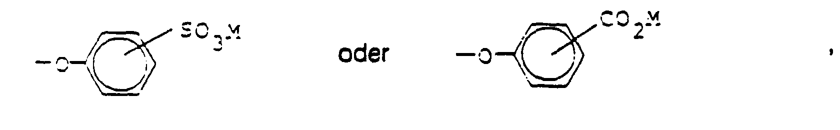 und
M Wasserstoff, ein Alkali- oder das Äquivalent eines Erdalkali-Ions bedeuten.

4. Verfahren zur Herstellung von Bleich-, Oxidations- oder Desinfektionsmittel enthaltend Verbindungen nach einem der Ansprüche 1 bis 3, dadurch gekennzeichnet, daß eine Imidocarbonsäure oder Sulfimidocarbonsäure der Formel 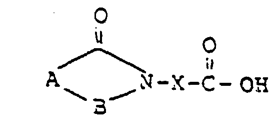 worin
A eine Gruppe der Formel 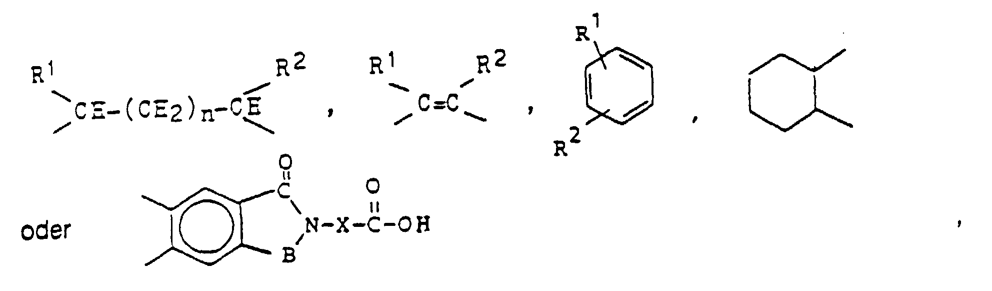
n die Zahlen 0, 1 oder 2,
R¹ Wasserstoff, Chlor, Brom, C₁-C₂₀-Alkyl, C₂-C₂₀-Alkenyl, Phenyl oder C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Phenylen
B eine Gruppe der Formeln C=O, SO₂,
bedeuten, gleichzeitig mit einem Gemisch aus einem kurzkettigen Carbonsäureanhydrid, wie Essigsäureanhydrid, Propionsäureanhydrid, Buttersäureanhydrid, bevorzugt Essigsäureanhydrid, und einem mit einer Carbonsäuregruppe oder Sulfonsäuregruppe substituierten Hydroxybenzolderivat und deren Salze in einem organischen Lösungsmittel im molaren Verhältnis 1-5:1-5:1, bevorzugt 1-3:1-3:1, umgesetzt werden.

5. Verfahren nach Anspruch 4, dadurch gekennzeichnet, daß als organische Lösungsmittel bevorzugt hochsiedende Kohlenwasserstoffe und/oder bevorzugt kurzkettige Carbonsäureanhydride verwendet werden.

6. Verfahren nach Anspruch 4 oder 5, dadurch gekennzeichnet, daß die Reaktionstemperatur zwischen 120°C und 300°C, bevorzugt zwischen 150°C und 250°C, liegt.

7. Verfahren nach einem der Ansprüche 4 bis 6, dadurch gekennzeichnet, daß als Katalysator ein Alkali- oder Erdalkalisalz einer Carbonsäure verwendet wird.

8. Verfahren zur Herstellung von Bleich-, Oxidations- oder Desinfektionsmittel enthaltend Verbindungen nach einem der Ansprüche 1 bis 3, dadurch gekennzeichnet, daß ein Imidocarbonsäurehalogenid oder Sulfimidocarbonsäurehalogenid der Formel 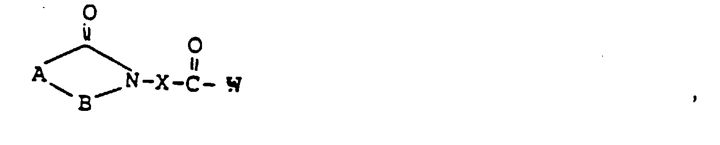 worin
A eine Gruppe der Formel 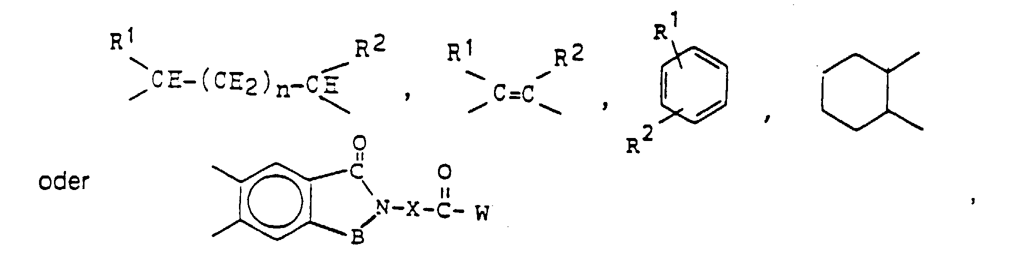
n die Zahlen 0, 1 oder 2,
R¹ Wasserstoff, Chlor, Brom, C₁-C₂₀-Alkyl, C₂-C₂₀-Alkenyl, Phenyl oder C₁-C₄-Alkylphenyl,
R² Wasserstoff, Chlor, Brom oder eine Gruppe der Formel -SO₃M, -CO₂M oder -OSO₃M,
X C₁-C₁₉-Alkylen oder Phenylen,
B eine Gruppe der Formeln C=O, SO₂,
W Fluor, Chlor, Brom, Jod, bevorzugt Chlor,
bedeuten, mit einem mit einer Carbonsäuregruppe oder Sulfonsäuregruppe substituierten Hydroxybenzolderivat und deren Salze im molaren Verhältnis 0,1-2,5:1, bevorzugt 0,5-1,5:1, in einem inerten, organischen, hochsiedenden Lösungsmittel bei einer Temperatur zwischen 80°C und 200°C umgesetzt werden.

9. Verfahren nach Anspruch 8, dadurch gekennzeichnet, daß als inerte, organische Lösungsmittel bevorzugt hochsiedende Kohlenwasserstoffe verwendet werden.

10. Verfahren nach Anspruch 8 oder 9, dadurch gekennzeichnet, daß die Reaktionstemperatur zwischen 80°C und 200°C, bevorzugt zwischen 100°C und 150°C, liegt.

11. Verfahren nach einem der Ansprüche 8 bis 10, dadurch gekennzeichnet, daß die Reaktionsdauer zwischen 60 und 360 min. liegt.

12. Verfahren zum Bleichen von textilen Gegenständen, dadurch gekennzeichnet, daß man Bleichmittel gemäß Anspruch 1 verwendet und der pH-Wert der verwendeten Waschlauge kleiner 10,3 ist, bevorzugt in einem Bereich von 8 bis 10,3 liegt.

## Claims (Claims for the following Contracting State(s): AT, BE, CH, DE, FR, GB, IT, LI, NL)

1. A persalt activator or salt thereof which is derived from an imidocarboxylic acid or sulfimidocarboxylic acid of the formula I: 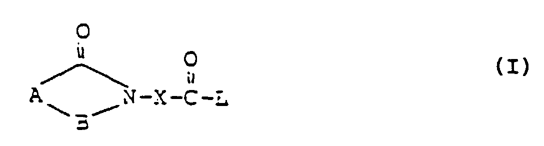 in which A is a group of the formula 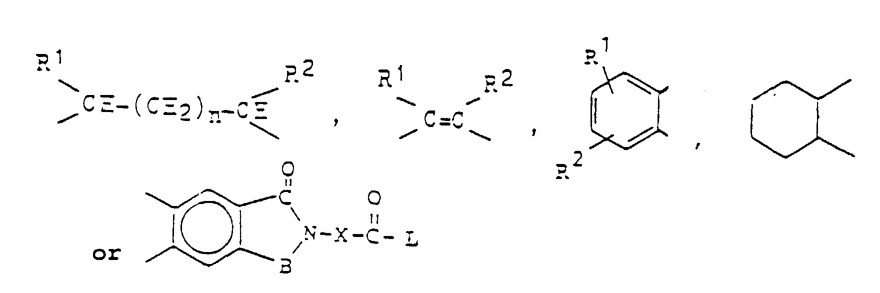
n is the number 0, 1 or 2,
R¹ is hydrogen, chlorine, bromine, C₁-C₂₀-alkyl, C₂-C₂₀-alkenyl, aryl, preferably phenyl, or alkylaryl, preferably C₁-C₄-alkylphenyl,
R² is hydrogen, chlorine, bromine or a group of the formula -SO₃M, -CO₂M or -OSO₃M,
X is C₁-C₁₉-alkylene or arylene, preferably phenylene,
B is a group of the formula C=O or SO₂,
L is a leaving group of the formula 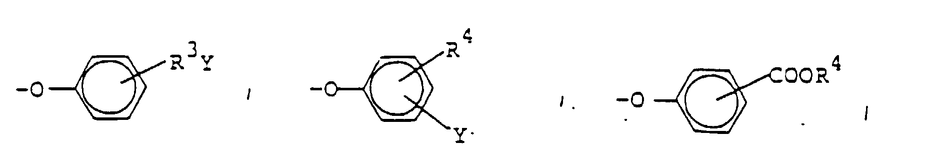 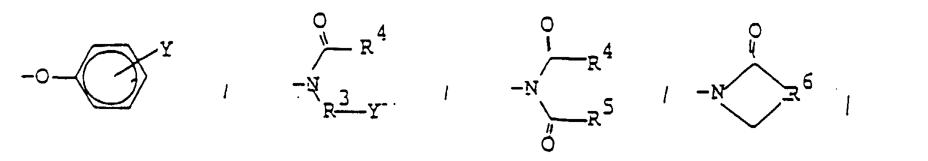 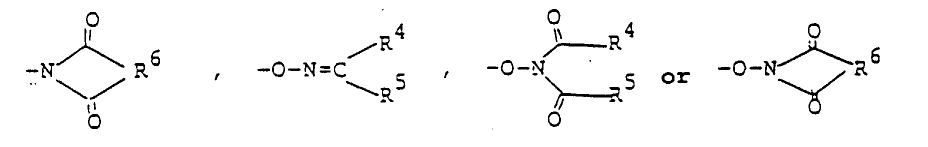 or a sugar residue,
R³ is C₁-C₁₉-alkylene,
R⁴ and R⁵ are C₁-C₂₀-alkyl,
R⁶ is C₁-C₁₉-alkylene or C₂-C₂₀-alkenylene,
Y is hydrogen, chlorine, bromine or a group of the formula -SO₃M, -CO₂M, -OSO₃M, -CONH₂, -N(R⁷)₃Z or -P(R⁷)₄Z,
R⁷ is C₁-C₃₀-alkyl,
Z is fluoride, chloride, bromide or iodide and
M is hydrogen, an alkali metal or ammonium ion or the equivalent of an alkaline earth metal ion,
with the proviso that
compounds of the formula I 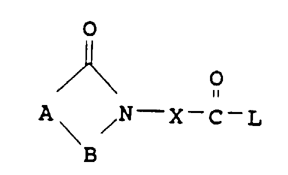 in which
A is a group of the formula 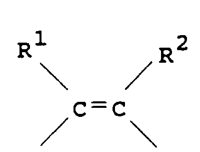
R¹ is hydrogen,
R² is hydrogen,
X is C₂-alkylene
L is a leaving group of the formula 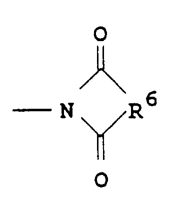 and
R⁶ is C₂-alkylene
and
compounds of the formula I 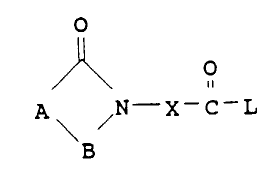 in which
A is a group of the formula 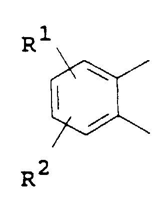
R¹ is hydrogen,
R² is hydrogen,
X is C₃-alkylene,
L is a leaving group of the formula 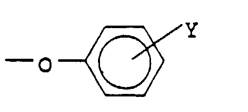 and
Y is p-chlorine,
are excepted.

2. A compound as claimed in claim 1, in which
A is a group of the formula -HC=CH-, 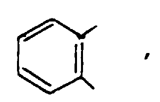 -CH₂-(CH₂)ₙ-CH₂-, 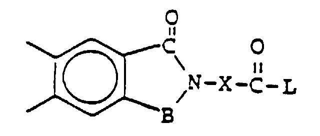 or -CH₂-CHR¹-,
n is the number 0 or 1,
R¹ is C₁-C₂₀-alkyl or C₂-C₂₀-alkenyl,
X is C₄-C₈-alkylene,
B is a group of the formula C=O or SO₂,
L is a leaving group of the formula 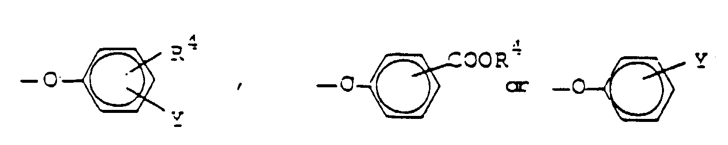
R⁴ is hydrogen or alkyl,
Y is hydrogen or a group of the formula -SO₃M, -CO₂M, -OSO₃M, -CONH₂, -N(R⁷)₃Z or -P(R⁷)₄Z,
R⁷ is C₁-C₄-alkyl, particularly preferably methyl,
Z is chloride and
M is hydrogen, an alkali metal or ammonium ion or the equivalent of an alkaline earth metal ion.

3. A compound as claimed in claim 1 or 2, in which
A is a group of the formula phenyl or -CH₂-CHR¹-,
R¹ is C₁-C₂₀-alkyl,
B is a group of the formula C=O or SO₂,
X is C₄-C₈-alkylene,
L is a leaving group of the formula 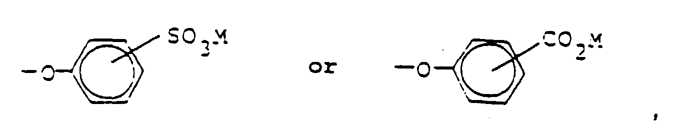 and
M is hydrogen, an alkali metal ion or the equivalent of an alkaline earth metal ion.

4. A process for the preparation of a compound as claimed in one of claims 1 to 3, which comprises reacting an imidocarboxylic acid or sulfimidocarboxylic acid of the formula 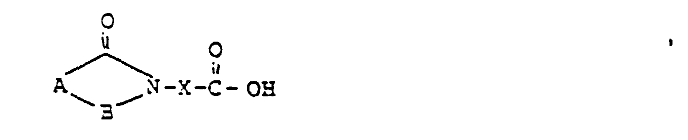 in which
A is a group of the formula 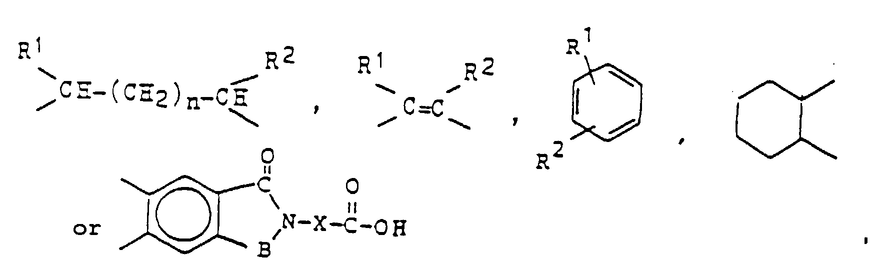
n is the number 0, 1 or 2,
R¹ is hydrogen, chlorine, bromine, C₁-C₂₀-alkyl, C₂-C₂₀-alkenyl, phenyl or C₁-C₄-alkylphenyl,
R² is hydrogen, chlorine, bromine or a group of the formula -SO₃M, -CO₂M or -OSO₃M,
X is C₁-C₁₉-alkylene or phenylene and
B is a group of the formula C=O or SO₂,
simultaneously with a mixture of a short-chain carboxylic acid anhydride, such as acetic anhydride, propionic anhydride or butyric anhydride, preferably acetic anhydride, and a hydroxybenzene derivative substituted by a carboxylic acid group or sulfonic acid group, or a salt thereof, in an organic solvent in a molar ratio of 1-5:1-5:1, preferably 1-3:1-3:1.

5. The process as claimed in claim 4, wherein the organic solvent used is preferably a highboiling hydrocarbon and/or preferably a short-chain carboxylic acid anhydride.

6. The process as claimed in claim 4 or 5, wherein the reaction temperature is between 120°C and 300°C, preferably between 150°C and 250°C.

7. The process as claimed in one of claims 4 to 6, wherein the catalyst used is an alkali metal salt or alkaline earth metal salt of a carboxylic acid.

8. A process for the preparation of a compound as claimed in one of claims 1 to 3, which comprises reacting an imidocarboxylic acid halide or sulfimidocarboxylic acid halide of the formula 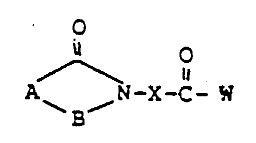 in which
A is a group of the formula 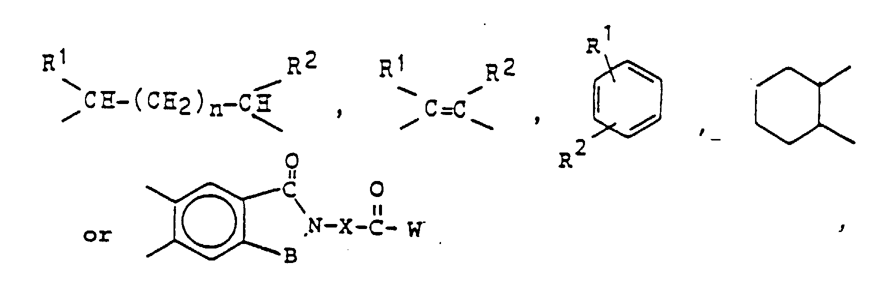
n is the number 0, 1 or 2,
R¹ is hydrogen, chlorine, bromine, C₁-C₂₀-alkyl, C₂-C₂₀-alkenyl, phenyl or C₁-C₄-alkylphenyl,
R² is hydrogen, chlorine, bromine or a group of the formula -SO₃M, -CO₂M or -OSO₃M,
X is C₁-C₁₉-alkylene or phenylene,
B is a group of the formula C=O or SO₂ and
W is fluorine, chlorine, bromine or iodine, preferably chlorine,
with a hydroxybenzene derivative substituted by a carboxylic acid group or sulfonic acid group, or a salt thereof, in a molar ratio of 0.1-2.5:1, preferably 0.5-1.5:1, in an inert, organic, highboiling solvent at a temperature between 80°C and 200°C.

9. The process as claimed in claim 8, wherein the inert, organic solvent used is a highboiling hydrocarbon.

10. The process as claimed in claim 8 or 9, wherein the reaction temperature is between 80°C and 200°C, preferably between 100°C and 150°C.

11. The process as claimed in one of claims 8 to 10, wherein the reaction time is between 60 and 360 minutes.

12. The use of a compound as claimed in claim 1 to 3 as a bleaching, oxidizing or disinfecting agent in detergents and cleaning agents.

13. A process for bleaching textile objects, which comprises using a compound as claimed in claim 1 as the bleaching agent and adjusting the pH of the wash liquor used to below 10.3, preferably in a range from 8 to 10.3.

## Claims (Claims for the following Contracting State(s): ES)

1. A bleaching, oxidizing or disinfecting agent containing a persalt activator or salt thereof which is derived from an imidocarboxylic acid or sulfimidocarboxylic acid of the formula I: 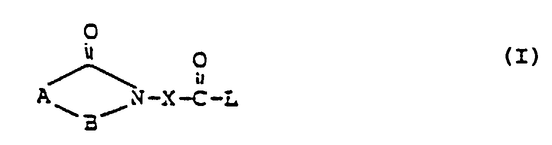 in which A is a group of the formula 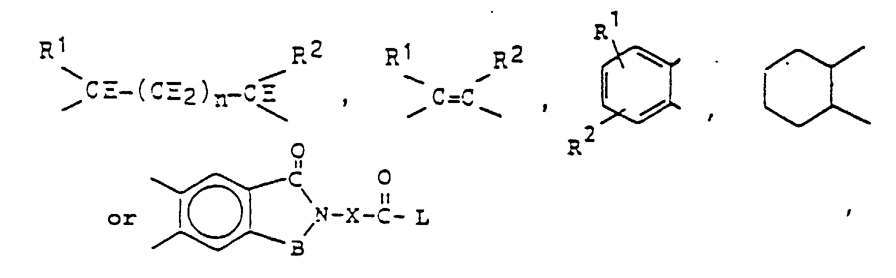
n is the number 0, 1 or 2,
R¹ is hydrogen, chlorine, bromine, C₁-C₂₀-alkyl, C₂-C₂₀-alkenyl, aryl, preferably phenyl, or alkylaryl, preferably C₁-C₄-alkylphenyl,
R² is hydrogen, chlorine, bromine or a group of the formula -SO₃M, -CO₂M or -OSO₃M,
X is C₁-C₁₉-alkylene or arylene, preferably phenylene,
B is a group of the formula C=O or SO₂,
L is a leaving group of the formula 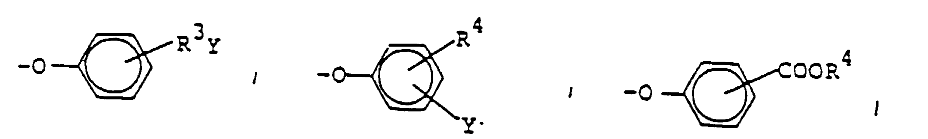 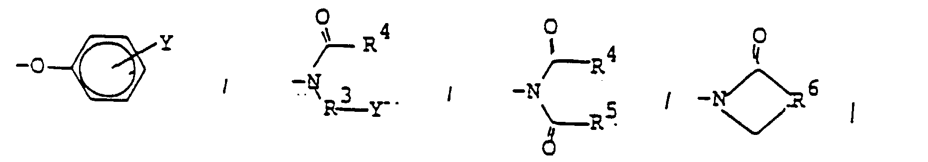 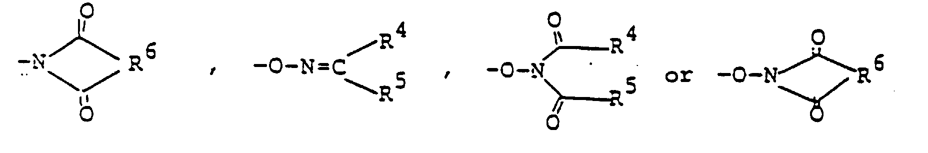 or a sugar residue,
R³ is C₁-C₁₉-alkylene,
R⁴ and R⁵ are C₁-C₂₀-alkyl,
R⁶ is C₁-C₁₉-alkylene or C₂-C₂₀-alkenylene,
Y is hydrogen, chlorine, bromine or a group of the formula -SO₃M, -CO₂M, -OSO₃M, -CONH₂, -N(R⁷)₃Z or -P(R⁷)₄Z,
R⁷ is C₁-C₃₀-alkyl,
Z is fluoride, chloride, bromide or iodide and
M is hydrogen, an alkali metal or ammonium ion or the equivalent of an alkaline earth metal ion,
with the proviso that
compounds of the formula I 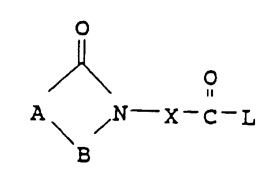 in which
A is a group of the formula 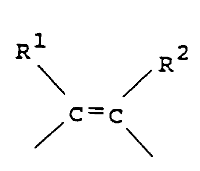
R¹ is hydrogen,
R² is hydrogen,
X is C₂-alkylene
L is a leaving group of the formula 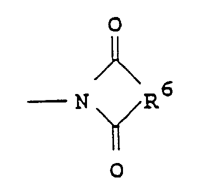 and
R⁶ is C₂-alkylene
and
compounds of the formula I 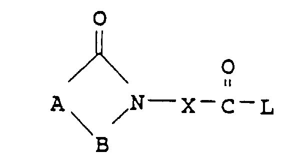 in which
A is a group of the formula 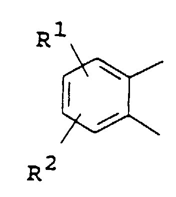
R¹ is hydrogen,
R² is hydrogen,
X is C₃-alkylene,
L is a leaving group of the formula 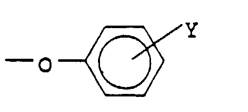 and
Y is p-chlorine,
are excepted.

2. A bleaching, oxidizing or disinfecting agent containing a compound as claimed in claim 1, in which
A is a group of the formula -HC=CH-, 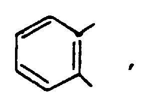 -CH₂-(CH₂)ₙ-CH₂-, 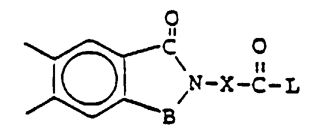 or -CH₂-CHR¹-,
n is the number 0 or 1,
R¹ is C₁-C₂₀-alkyl or C₂-C₂₀-alkenyl,
X is C₄-C₈-alkylene,
B is a group of the formula C=O or SO₂,
L is a leaving group of the formula 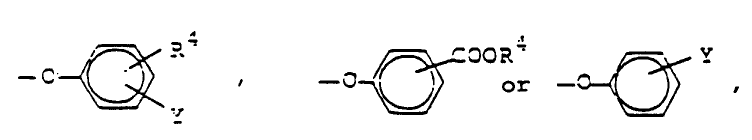
R⁴ is hydrogen or alkyl,
Y is hydrogen or a group of the formula -SO₃M,--CO₂M, -OSO₃M, -CONH₂, -N(R⁷)₃Z or -P(R⁷)₄Z,
R⁷ is C₁-C₄-alkyl, particularly preferably methyl,
Z is chloride and
M is hydrogen, an alkali metal or ammonium ion or the equivalent of an alkaline earth metal ion.

3. A bleaching, oxidizing or disinfecting agent containing a compound as claimed in claim 1 or 2, in which
A is a group of the formula phenyl or -CH₂-CHR¹-,
R¹ is C₁-C₂₀-alkyl,
B is a group of the formula C=O or SO₂,
X is C₄-C₈-alkylene,
L is a leaving group of the formula 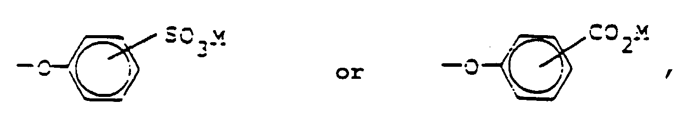 and
M is hydrogen, an alkali metal ion or the equivalent of an alkaline earth metal ion.

4. A process for the preparation of a bleaching, oxidizing or disinfecting agent containing a compound as claimed in one of claims 1 to 3, which comprises reacting an imidocarboxylic acid or sulfimidocarboxylic acid of the formula 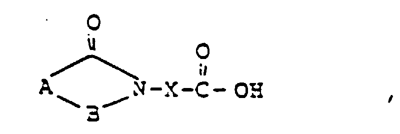 in which
A is a group of the formula 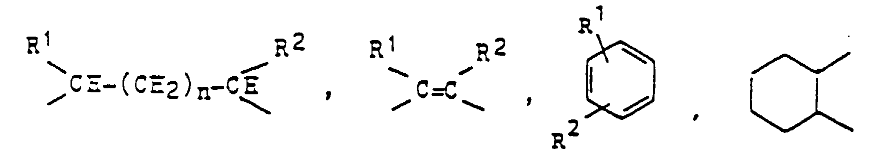 or 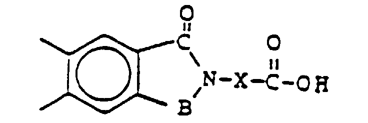
n is the number 0, 1 or 2,
R¹ is hydrogen, chlorine, bromine, C₁-C₂₀-alkyl, C₂-C₂₀-alkenyl, phenyl or C₁-C₄-alkylphenyl,
R² is hydrogen, chlorine, bromine or a group of the formula -SO₃M, -CO₂M or -OSO₃M,
X is C₁-C₁₉-alkylene or phenylene and
B is a group of the formula C=O or SO₂,
simultaneously with a mixture of a short-chain carboxylic acid anhydride, such as acetic anhydride, propionic anhydride or butyric anhydride, preferably acetic anhydride, and a hydroxybenzene derivative substituted by a carboxylic acid group or sulfonic acid group, or a salt thereof, in an organic solvent in a molar ratio of 1-5:1-5:1, preferably 1-3:1-3:1.

5. The process as claimed in claim 4, wherein the organic solvent used is preferably a highboiling hydrocarbon and/or preferably a short-chain carboxylic acid anhydride.

6. The process as claimed in claim 4 or 5, wherein the reaction temperature is between 120°C and 300°C, preferably between 150°C and 250°C.

7. The process as claimed in one of claims 4 to 6, wherein the catalyst used is an alkali metal salt or alkaline earth metal salt of a carboxylic acid.

8. A process for the preparation of a bleaching, oxidizing or disinfecting agent containing a compound as claimed in one of claims 1 to 3, which comprises reacting an imidocarboxylic acid halide or sulfimidocarboxylic acid halide of the formula 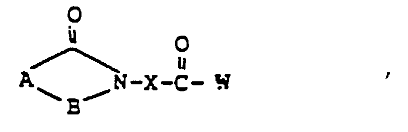 in which
A is a group of the formula 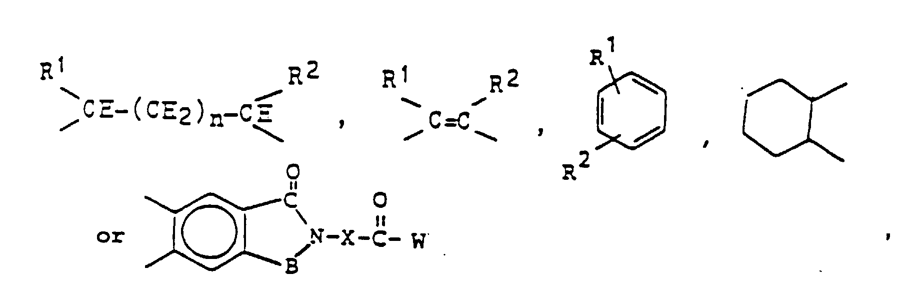
n is the number 0, 1 or 2,
R¹ is hydrogen, chlorine, bromine, C₁-C₂₀-alkyl, C₂-C₂₀-alkenyl, phenyl or C₁-C₄-alkylphenyl,
R² is hydrogen, chlorine, bromine or a group of the formula -SO₃M, -CO₂M or -OSO₃M,
X is C₁-C₁₉-alkylene or phenylene,
B is a group of the formula C=O or SO₂ and
W is fluorine, chlorine, bromine or iodine, preferably chlorine,
with a hydroxybenzene derivative substituted by a carboxylic acid group or sulfonic acid group, or a salt thereof, in a molar ratio of 0.1-2.5:1, preferably 0.5-1.5:1, in an inert, organic, highboiling solvent at a temperature between 80°C and 200°C.

9. The process as claimed in claim 8, wherein the inert, organic solvent used is preferably a highboiling hydrocarbon.

10. The process as claimed in claim 8 or 9, wherein the reaction temperature is between 80°C and 200°C, preferably between 100°C and 150°C.

11. The process as claimed in one of claims 8 to 10, wherein the reaction time is between 60 and 360 minutes.

12. A process for bleaching textile objects, which comprises using a bleaching agent as claimed in claim 1 and adjusting the pH of the wash liquor used to below 10.3, preferably in a range from 8 to 10.3.

## Revendications (Revendications pour l'(les) Etat(s) contractant(s) suivant(s): AT, BE, CH, DE, FR, GB, IT, LI, NL)

1. Activateurs de type persel et leurs sels, qui dérivent des acides imidocarboxyliques et des acides sulfimidocarboxyliques de formule générale I : 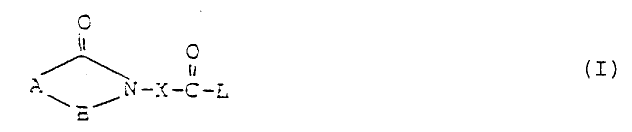 où A représente un groupe de formule 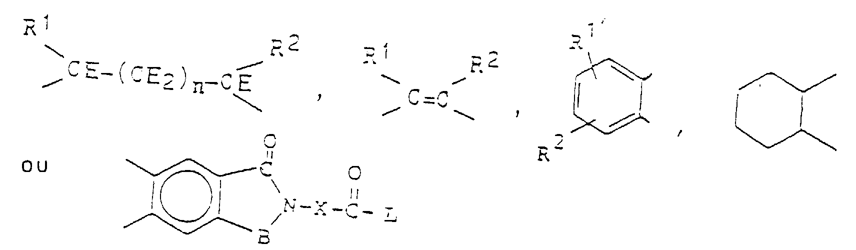
n vaut 0, 1 ou 2,
R¹ représente un atome d'hydrogène, un atome de chlore, un atome de brome, des groupes alkyle en C₁-C₂₀, alcényle en C₂-C₂₀, aryle, de préférence phényle ou alkylaryle, de préférence (alkyle en C₁-C₄)phényle,
R² représente un atome d'hydrogène, un atome de chlore, un atome de brome, ou un groupe de formule -SO₃M, -CO₂M ou -OSO₃M,
X représente un groupe alkylène en C₁-C₁₉, ou arylène, de préférence phénylène,
B représente un groupe de formule C=O, SO₂,
L représente un groupe partant de formule 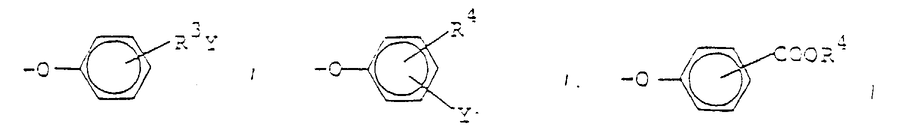 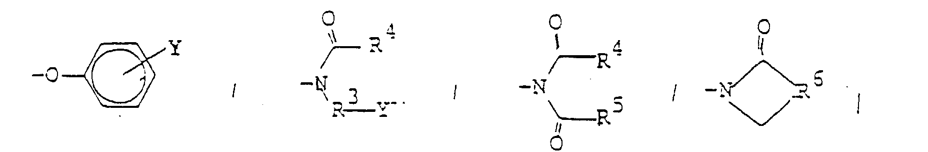 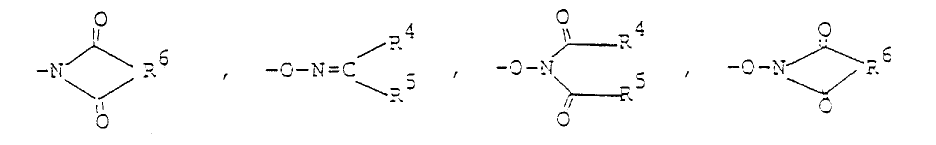 ou un reste sucre,
R³ représente un groupe alkylène en C₁-C₁₉,
R⁴ et R⁵ représentent un groupe alkyle en C₁-C₂₀,
R⁶ représente des groupes alkylène en C₁-C₁₉, alcénylène en C₂-C₂₀,
Y représente un atome d'hydrogène, un atome de chlore, un atome de brome ou un groupe de formules -SO₃M, -CO₂M, -OSO₃M, -CONH₂, -N(R⁷)₃Z ou -P(R⁷)₄Z,
R⁷ représente un groupe alkyle en C₁-C₃₀,
Z représente un fluorure, un chlorure, un bromure ou un iodure et
M représente un atome d'hydrogène, un ion alcalin ou ammonium ou l'équivalent d'un ion alcalino-terreux,
à la condition que des composés de formule générale I 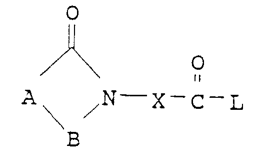 dans laquelle
A représente un groupe de formule 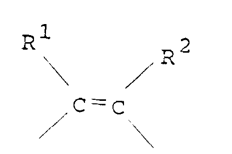
R¹ représente un atome d'hydrogène,
R² représente un atome d'hydrogène,
X représente un groupe alkylène en C₂,
L représente un groupe partant de formule 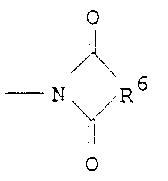 et
R⁶ représente un groupe alkylène en C₂
et
des composés de formule générale I 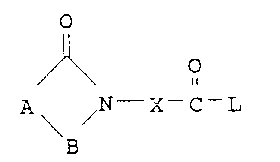 dans laquelle
A représente un groupe de formule 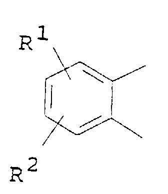
R¹ représente un atome d'hydrogène,
R² représente un atome d'hydrogène,
X représente un groupe alkylène en C₃,
L représente un groupe partant de formule 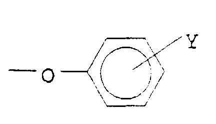 et
Y représente p-chlore,
soient exclus.

2. Composés selon la revendication 1, dans laquelle
A représente un groupe de formule -HC=CH-, 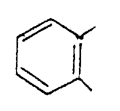 -CH₂-(CH₂)ₙ-CH₂- 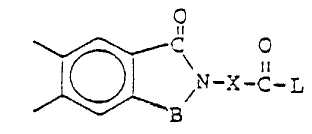 ou -CH₂-CHR¹-,
n vaut 0 ou 1,
R¹ représente des groupes alkyle en C₁-C₂₀ ou alcényle en C₂-C₂₀,
X représente alkylène en C₄-C₈,
B représente un groupe de formules C=O, SO₂,
L représente un groupe partant de formule 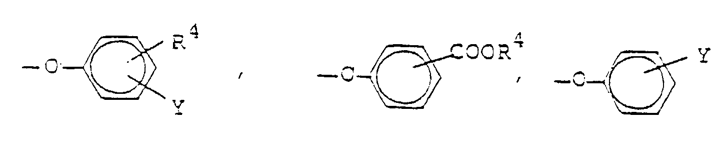
R⁴ représente un atome d'hydrogène, un groupe alkyle,
Y représente un atome d'hydrogène ou un groupe de formules -SO₃M, -CO₂M, -OSO₃M, -CONH₂, -N(R⁷)₃Z ou -P(R⁷)₄Z,
R⁷ représente un groupe alkyle en C₁-C₄, de préférence particulièrement méthyle,
Z représente chlorure, et
M représente un atome d'hydrogène, un ion alcalin ou ammonium ou l'équivalent d'un ion alcalino-terreux.

3. Composés selon la revendication 1 ou 2, où
A représente un groupe de formule phényle, -CH₂CHR¹-,
R¹ représente un groupe alkyle en C₁-C₂₀,
B représente un groupe de formule -C=O, SO₂,
X représente un groupe alkylène en C₄-C₈,
L représente un groupe partant de formule 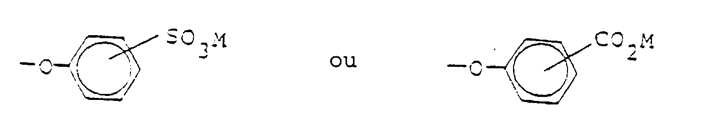 et
M représente un atome d'hydrogène, un ion alcalin ou l'équivalent d'un ion alcalino-terreux.

4. Procédé pour la préparation des composés selon l'une des revendications 1 à 3, caractérisé en ce que l'on fait réagir un acide imidocarboxylique ou sulfimidocarboxylique de formule 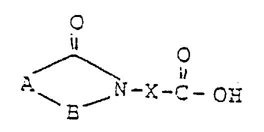 dans laquelle
A représente un groupe de formule 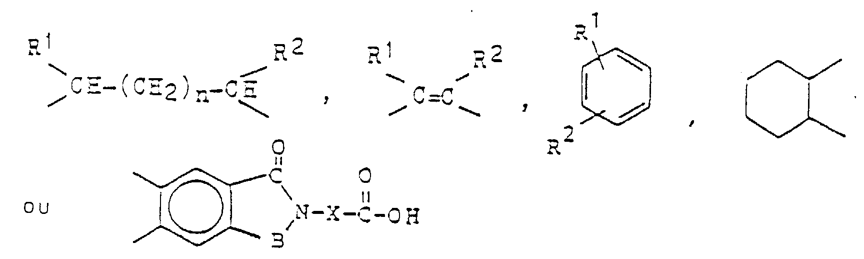
n vaut 0, 1 ou 2,
R¹ représente un atome d'hydrogène, un atome de chlore, un atome de brome, des groupes alkyle en C₁-C₂₀, alcényle en C₂-C₂₀, phényle ou (alkyle en C₁-C₄)phényle,
R² représente un atome d'hydrogène, un atome de chlore, un atome de brome ou un groupe de formule -SO₃M, -CO₂M, ou -OSO₃M,
X représente un groupe alkylène en C₁-C₁₉ ou phénylène,
B représente un groupe de formule C=O, SO₂,
simultanément avec un mélange d'un anhydride d'acide carboxylique à chaîne courte, tel que l'anhydride acétique, l'anhydride propionique, l'anhydride butyrique, de préférence l'anhydride acétique, et un dérivé d'hydroxybenzène substitué par un groupe acide carboxylique ou acide sulfonique et leurs sels, dans un solvant organique dans un rapport molaire de 1 à 5:1 à 5:1, de préférence 1 à 3:1 à 3:1.

5. Procédé selon la revendication 4, caractérisé en ce que l'on utilise comme solvants organiques de préférence des hydrocarbures à haut point d'ébullition et/ou de préférence des anhydrides d'acides carboxyliques à chaîne courte.

6. Procédé selon la revendication 4 ou 5, caractérisé en ce que la température réactionnelle est comprise entre 120 et 300 °C, de préférence entre 150 et 250 °C.

7. Procédé selon l'une des revendications 4 à 6, caractérisé en ce que l'on utilise comme catalyseur un sel de métal alcalin ou alcalino-terreux d'un acide carboxylique.

8. Procédé de préparation de composés selon l'une des revendications 1 à 3, caractérisé en ce que l'on fait réagir un halogénure d'acide imidocarboxylique ou un halogénure d'acide sulfimidocarboxylique de formule 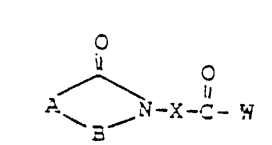 dans laquelle
A représente un groupe de formule 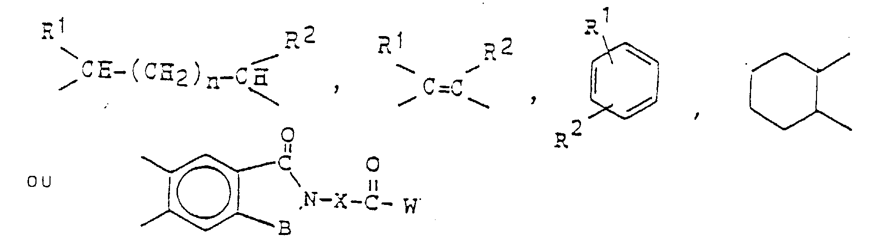
n vaut 0, 1 ou 2,
R¹ représente un atome d'hydrogène, un atome de chlore, un atome de brome, des groupes alkyle en C₁-C₂₀, alcényle en C₂-c₂₀, phényle ou (alkyl en C₁-C₄)-phényle,
R² représente un atome d'hydrogène, un atome de chlore, un atome de brome ou un groupe de formule -SO₃M, -CO₂M ou -OSO₃M,
X représente un groupe alkylène en C₁-C₁₉ ou phénylène,
B représente un groupe de formule C=O, SO₂,
W représente un atome de fluor, de chlore, de brome, d'iode, de préférence de chlore,
avec un dérivé d'hydroxybenzène substitué par un groupe acide carboxylique ou acide sulfonique, et leurs sels dans un rapport molaire de (0,1 à 2,5):1, de préférence de (0,5 à 1,5):1, dans un solvant organique inerte à haut point d'ébullition, à une température comprise entre 80 et 200 °C.

9. Procédé selon la revendication 8, caractérisé en ce que l'on utilise comme solvant organique inerte de préférence des hydrocarbures à haut point d'ébullition.

10. Procédé selon la revendication 8 ou 9, caractérisé en ce que la température réactionnelle est comprise entre 80 et 200 °C, de préférence entre 100 et 150 °C.

11. Procédé selon l'une des revendications 8 à 10, caractérisé en ce que la durée réactionnelle est comprise entre 60 et 360 minutes.

12. Utilisation des composés selon l'une des revendications 1 à 3 en tant qu'agents blanchissants, oxydants et désinfectants dans les détergents et les produits de nettoyage.

13. Procédé pour le blanchissage de produits textiles, caractérisé en ce que l'on utilise en tant que composés blanchissants des composés selon la revendication 1 et la valeur du pH du bain de lavage utilisé est inférieure à 10,3, de préférence dans un domaine de 8 à 10,3.

## Revendications (Revendications pour l'(les) Etat(s) contractant(s) suivant(s): ES)

1. Agents blanchissants, oxydants et désinfectants, contenant des activateurs de type persel et leurs sels, qui dérivent des acides imidocarboxyliques et des acides sulfimidocarboxyliques de formule générale I : 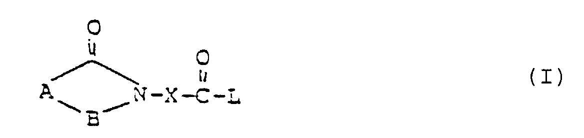 où A représente un groupe de formule 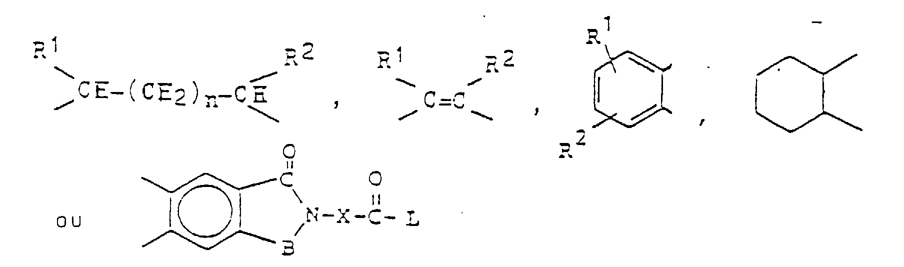
n vaut 0, 1 ou 2,
R¹ représente un atome d'hydrogène, un atome de chlore, un atome de brome, des groupes alkyle en C₁-C₂₀, alcényle en C₂-C₂₀, aryle, de préférence phényle, ou alkylaryle, de préférence (alkyl en C₁-C₄)phényle,
R² représente un atome d'hydrogène, un atome de chlore, un atome de brome, ou un groupe de formule -SO₃M, -CO₂M ou -OSO₃M,
X représente un groupe alkylène en C₁-C₁₉ ou arylène, de préférence phénylène,
B représente un groupe de formules C=O, SO₂,
L représente un groupe partant de formule 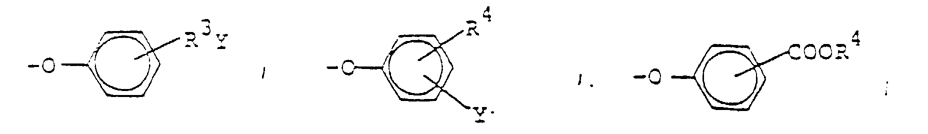 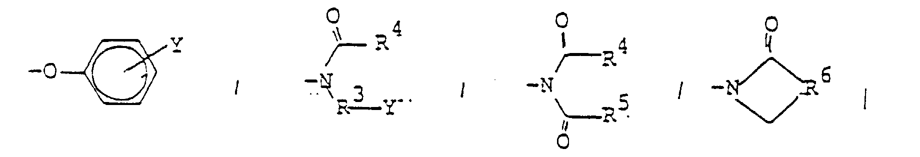 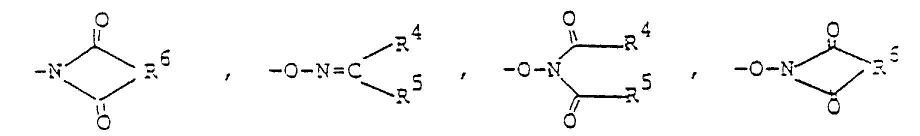 ou un reste sucre,
R³ représente un groupe alkylène en C₁-C₁₉,
R⁴ et R⁵ représentent un groupe alkyle en C₁-C₂₀,
R⁶ représente des groupes alkylène en C₁-C₁₉, alcénylène en C₂-C₂₀,
Y représente un atome d'hydrogène, un atome de chlore, un atome de brome ou un groupe de formule -SO₃M, -CO₂M, -OSO₃M, -CONH₂, -N(R⁷)₃Z ou -P(R⁷)₄Z,
R⁷ représente un groupe alkyle en C₁-C₃₀,
Z représente un fluorure, un chlorure, un bromure ou un iodure et
M représente un atome d'hydrogène, un ion alcalin ou ammonium ou l'équivalent d'un ion alcalino-terreux,
à la condition que des composés de formule générale I 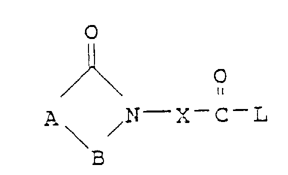 dans laquelle
A représente un groupe de formule 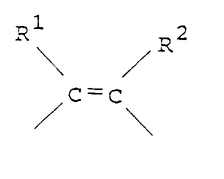
R¹ représente un atome d'hydrogène,
R² représente un atome d'hydrogène,
X représente un groupe alkylène en C₂,
L représente un groupe partant de formule 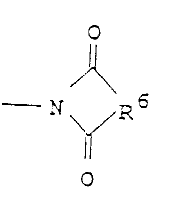 et
R⁶ représente un groupe alkylène en C₂
et
des composés de formule générale I 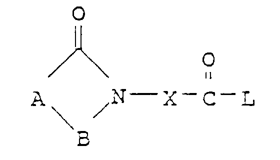 dans laquelle
A représente un groupe de formule 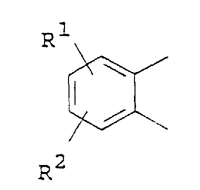
R¹ représente un atome d'hydrogène,
R² représente un atome d'hydrogène,
X représente un groupe alkylène en C₃,
L représente un groupe partant de formule 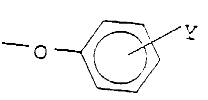 et
Y représente p-chlore,
soient exclus.

2. Agents blanchissants, oxydants ou désinfectants contenant des composés selon la revendication 1, dans laquelle
A représente un groupe de formule -HC=CH-, 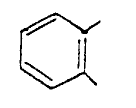 -CH₂-(CH₂)ₙ-CH₂- 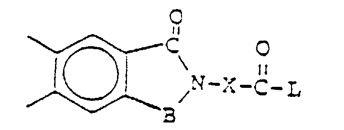 ou -CH₂-CHR¹-,
n vaut 0 ou 1,
R¹ représente des groupes alkyle en C₁-C₂₀ ou alcényle en C₂-C₂₀,
X représente alkylène en C₄-C₈,
B représente un groupe de formules C=O, SO₂,
L représente un groupe partant de formule 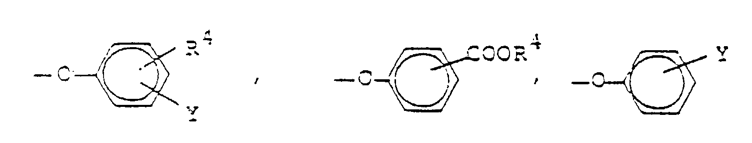
R⁴ représente un atome d'hydrogène, un groupe alkyle,
Y représente un atome d'hydrogène ou un groupe de formules -SO₃M, -CO₂M, -OSO₃M, -CONH₂, -N(R⁷)₃Z ou -P(R⁷)₄Z,
R⁷ représente un groupe alkyle en C₁-C₄, de préférence en particulier méthyle,
Z représente un chlorure et
M représente un atome d'hydrogène, un ion alcalin ou ammonium ou l'équivalent d'un ion alcalino-terreux.

3. Agents blanchissants, oxydants ou désinfectants contenant des composés selon la revendication 1 ou 2, où
A représente un groupe de formule, phényle, -CH₂CHR¹-,
R¹ représente un groupe alkyle en C₁-C₂₀,
B représente un groupe de formule C=O, SO₂,
X représente un groupe alkylène en C₄-C₈,
L représente un groupe partant de formule 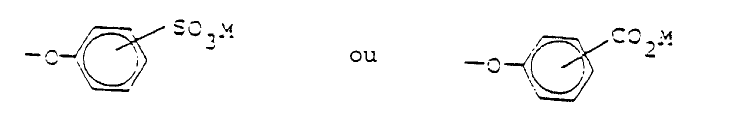 et
M représente un atome d'hydrogène ou un ion alcalin ou l'équivalent d'un ion alcalino-terreux.

4. Procédé pour la préparation d'agents blanchissants, oxydants ou désinfectants contenant des composés selon l'une des revendications 1 à 3, caractérisé en ce que l'on fait réagir un acide imidocarboxylique ou sulfimidocarboxylique de formule 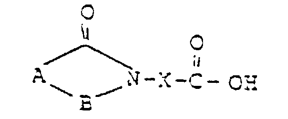 dans laquelle
A représente un groupe de formule 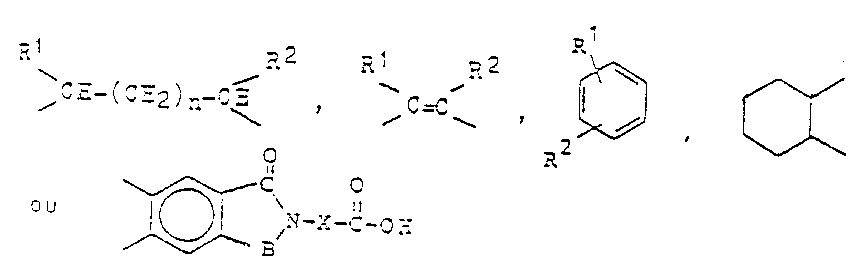
n vaut 0, 1 ou 2,
R¹ représente un atome d'hydrogène, un atome de chlore, un atome de brome, des groupes alkyle en C₁-C₂₀, alcényle en C₂-C₂₀, phényle ou (alkyl en C₁-C₄)-phényle,
R² représente un atome d'hydrogène, un atome de chlore, un atome de brome ou un groupe de formule -SO₃M, -CO₂M ou -OSO₃M,
X représente un groupe alkylène en C₁-C₁₉ ou phénylène,
B représente un groupe de formule C=O, SO₂,
simultanément avec un mélange d'un anhydride d'acide carboxylique à chaîne courte, tel que l'anhydride acétique, l'anhydride propionique, l'anhydride butyrique, de préférence l'anhydride acétique, et un dérivé d'hydroxybenzène substitué par un groupe acide carboxylique ou acide sulfonique et leurs sels, dans un solvant organique dans un rapport molaire de (1 à 5):(1 à 5):1, de préférence (1 à 3):(1 à 3):1.

5. Procédé selon la revendication 4, caractérisé en ce que l'on utilise comme solvants organiques de préférence des hydrocarbures à haut point d'ébullition et/ou de préférence des anhydrides d'acides carboxyliques à chaîne courte.

6. Procédé selon la revendication 4 ou 5, caractérisé en ce que la température réactionnelle est comprise entre 120 et 300 °C, de préférence entre 150 et 250 °C.

7. Procédé selon l'une des revendications 4 à 6, caractérisé en ce que l'on utilise comme catalyseur un sel de métal alcalin ou alcalino-terreux d'un acide carboxylique.

8. Procédé de préparation d'agents blanchissants, oxydants ou désinfectants contenant des composés selon l'une des revendications 1 à 3, caractérisé en ce que l'on fait réagir un halogénure d'acide imidocarboxylique ou un halogénure d'acide sulfimidocarboxylique de formule 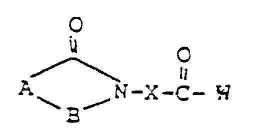 dans laquelle
A représente un groupe de formule 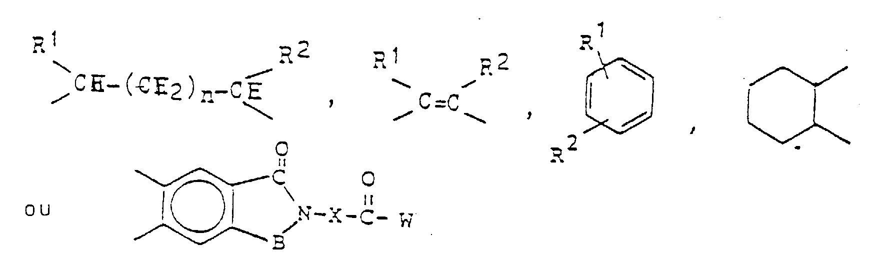
n vaut 0, 1 ou 2,
R¹ représente un atome d'hydrogène, un atome de chlore, un atome de brome, des groupes alkyle en C₁-C₂₀, alcényle en C₂-C₂₀, phényle ou (alkyl en C₁-C₄)phényle,
R² représente un atome d'hydrogène, un atome de chlore, un atome de brome ou un groupe de formule -SO₃M, -CO₂M ou -OSO₃M,
X représente un groupe alkylène en C₁-C₁₉ ou phénylène,
B représente un groupe de formules C=O, SO₂,
W représente un atome de fluor, de chlore, de brome, d'iode, de préférence de chlore,
avec un dérivé d'hydroxybenzène substitué par un groupe acide carboxylique ou acide sulfonique et leurs sels, dans un rapport molaire de (0,1 à 2,5):1, de préférence de (0,5 à 1,5):1, dans un solvant organique inerte à haut point d'ébullition à une température comprise entre 80 et 200 °C.

9. Procédé selon la revendication 8, caractérisé en ce que l'on utilise comme solvant organique inerte de préférence des hydrocarbures à haut point d'ébullition.

10. Procédé selon la revendication 8 ou 9, caractérisé en ce que la température réactionnelle est comprise entre 80 et 200 °C, de préférence entre 100 et 150 °C.

11. Procédé selon l'une des revendications 8 à 10, caractérisé en ce que la durée réactionnelle est comprise entre 60 et 360 minutes.

12. Procédé pour le blanchissage de produits textiles, caractérisé en ce que l'on utilise en tant que composés blanchissants des composés selon la revendication 1 et la valeur du pH du bain de lavage utilisé est inférieure à 10,3, de préférence dans un domaine de 8 à 10,3.
